# EUROPEAN PATENT SPECIFICATION

(11) **EP 3 312 895 B1**
(45) Date of publication and mention of the grant of the patent: **28.07.2021**
(21) Application number: 16195374.0
(22) Date of filing: 24.10.2016
(51) Int. Cl.: H01L 51/50, C07D 221/18

(54) **ORGANIC SEMICONDUCTING MATERIAL COMPRISING AN ELECTRICAL N-DOPANT AND AN ELECTRON TRANSPORT MATRIX AND ELECTRONIC DEVICE COMPRISING THE SEMICONDUCTING MATERIAL**
ORGANISCHES HALBLEITERMATERIAL MIT EINEM ELEKTRISCHEN N-DOTIERSTOFF SOWIE ELEKTRONENTRANSPORTMATRIX UND ELEKTRONISCHE VORRICHTUNG MIT DEM HALBLEITERMATERIAL
MATÉRIAU ORGANIQUE SEMI-CONDUCTEUR COMPRENANT UN DOPANT-N ÉLECTRIQUE ET UNE MATRICE DE TRANSPORT D'ÉLECTRONS ET DISPOSITIF ÉLECTRONIQUE COMPRENANT LE MATÉRIAU SEMI-CONDUCTEUR

(43) Date of publication of application: 25.04.2018
(73) Proprietor: Novaled GmbH, 01099 Dresden (DE); Samsung SDI Co., Ltd., Yongin-si, Gyeonggi-do 17084 (KR)
(72) Inventor: ROTHE, Carsten, D-01307 Dresden (DE); PAVICIC, Domagoj, D-01307 Dresden (DE); GANIER, Jerome, D-01307 Dresden (DE); JANKUS, Vygintas, D-01307 Dresden (DE); KIM, Hyungsun, Gyeonggi-do, 443-803 (KR); KIM, Byungku, Gyeonggi-do, 443-803 (KR)
(74) Representative: Michalski Hüttermann & Partner Patentanwälte mbB

(56) References cited:
- EP-A1- 2 999 019
- WO-A1-2013/079217
- WO-A1-2016/171358
- US-A1- 2003 039 858
- HWANGYU SHIN ET AL: "Synthesis and electroluminescence property of new hexaphenylbenzene derivatives including amine group for blue emitters", NANOSCALE RESEARCH LETTERS, vol. 8, no. 1, 1 January 2013 (2013-01-01) , page 421, XP55324498, ISSN: 1556-276X, DOI: 10.1021/cm040081o

## Description

### Technical Field

The present invention relates to an organic semiconducting material and to an electronic device comprising the semiconducting material, particularly to an electroluminescent device, particularly to an organic light emitting diode (OLED), wherein the semiconducting material comprises a first electron transport matrix compound and an electrical n-dopant; the invention pertains also to a device comprising the electric device and/or the electroluminescent device, particularly to a display device, particularly to a display device comprising the OLED.

### Background Art

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer HTL, an emission layer EML, an electron transport layer ETL, and a cathode, which are sequentially stacked on a substrate. In this regard, the HTL, the EML, and the ETL are thin films formed from organic compounds.

US 2003/039858 A1 refers to a light-emitting device comprising a pair of electrodes and one or more organic layers disposed therebetween, one or more organic layers comprising a light-emitting layer, wherein the light-emitting device utilizes a triplet exciton for light emission and comprises at least one compound represented by the following formula (1): 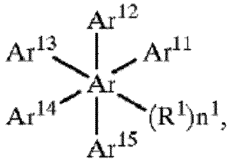 wherein each of Ar11, Ar12, Ar13, Ar14 and Ar15 represents an aryl group or a heteroaryl group; Ar represents an aryl group; R1 represents a substituent; and n1 represents an integer of 0 or more.

EP 2 999 019 A1 refers to an organic light-emitting diode comprising an emission layer and an electron transport layer stack of at least two electron transport layers, wherein a first electron transport layer and a second electron transport layer comprise at least one matrix compound and in addition,
- the at least first electron transport layer comprises a lithium halide or a lithium organic complex; and the at least second electron transport layer comprises an elemental metal selected from the group of lithium, magnesium and/or ytterbium; or
- the at least first electron transport layer comprises an elemental metal selected from the group of lithium, magnesium and/or ytterbium; and the at least second electron transport layer comprises a lithium halide or a lithium organic complex; wherein the electron transport layer or layers comprising a lithium halide or a lithium organic complex is free of an elemental metal, and the electron transport layer or layers that comprises an elemental metal is free of a metal salt and/or a metal organic complex.

HWANGYU SHIN ET AL, "Synthesis and electroluminescence property of new hexaphenylbenzene derivatives including amine group for blue emitters", NANOSCALE RESEARCH LETTERS, (20130101), vol. 8, no. 1, doi:10.1021/cm040081o, ISSN 1556-276X, page 421.

WO 2016/171358 A1 refers to a compound for an organic optoelectric device, an organic optoelectric device, and a display device are disclosed.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. There is continuing demand for development of improved materials, with the aim that operational voltage is as low as possible while brightness/luminance is high, and that injection and flow of holes and electrons is balanced, so that an OLED having the above-described structure has excellent efficiency and/or a long lifetime.

One of well-established approaches for achieving low operational voltages and high current densities/luminances is electrical p- and/or n-doping in charge injection/charge transport layers, and especially redox doping which generates doped layers with high charge carrier concentrations. In a previous application PCT-KR2015-012551, some of the authors of the present application developed new electron transport matrix compound combining bulky aromatic groups with properly designed electron transport units and successfully proved the inventive electron transport matrix compound in electrically undoped layers of OLED devices. To enable further increase in device performance, the present invention implements the inventive charge transport compounds in a redox-doped semiconducting material, and further implements the inventive semiconducting material in electronic devices, e.g. as electron transport layer in OLEDs.

### DISCLOSURE

Aspects of the present invention provide an organic semiconducting material for an electronic device, particularly for a light-emitting device comprising an emission layer and at least two electrodes, for increasing the efficiency, such as the external quantum efficiency EQE, and for achieving low operating voltage and long lifetime, particularly in top and/or bottom emission organic light-emitting diodes (OLEDs).

Another aspect of the present invention provides an electronic device comprising the semiconducting material, particularly an electroluminescent device. Still another aspect of the present invention provides a display device comprising the electroluminescent device. According to an aspect of the present invention, there is provided organic semiconducting material comprising at least one electron transport matrix and at least one electrical n-dopant, wherein the electron transport matrix comprises at least one first matrix compound according to Chemical Formula I: 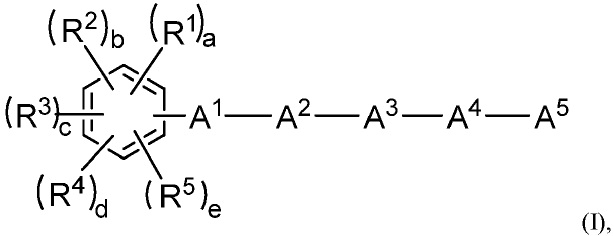 wherein
- A¹, A², A³ and A⁴: is independently selected from single bond, an unsubstituted or substituted C₆ to C₃₀ arylene and an unsubstituted or substituted C₁ to C₃₀ heteroarylene;
- A⁵: is selected from an unsubstituted or substituted C₆ to C₄₀ aryl group and/or from an unsubstituted or substituted C₂ to C₄₀ heteroaryl group;
- R¹ to R⁵: are independently a substituted or unsubstituted C₆ to C₃₀ aryl group, a substituted or unsubstituted C₂ to C₃₀ heteroaryl group;
- a to e: are independently an integer of 0 or 1 and 4 ≤ a+b+c+d+e ≤ 5;
wherein, in formula (I), in the substituted group, at least one hydrogen is replaced by
(i) deuterium,
(ii) a halogen,
(iii) a C₂ to C₆₀ tertiary amino group, wherein the nitrogen atom of the tertiary amino group is substituted with two independently selected C₁ to C₃₀ hydrocarbyl groups or the nitrogen atom of the C₂ to C₆₀ tertiary amino group_forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(v) a C₁ to C₂₂ silyl group,
(vi) a C₁ to C₃₀ alkyl group,
(vii) a C₁ to C₁₀ alkylsilyl group,
(viii) a C₆ to C₂₂ arylsilyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

In the present specification "A¹, A², A³ and A⁴ is independently selected from single bond" means that if "A¹, A², A³ and A⁴" are selected to be a single bond, "A¹, A², A³ and A⁴" forms together one single bond.

In the present specification "A¹, A², A³ and A⁴ is independently selected from single bond" means that if at least two directly connected members thereof, for example "A¹, A²", are selected to be a single bond, these connected members forms together one single bond.

In the present specification "A¹, A², A³ and A⁴ is independently selected from single bond" means that if at least three directly connected members thereof, for example "A², A³, A⁴ ", are selected to be a single bond, these directly connected members forms together one single bond.

In the present specification, the term "wherein in the substituted group, at least one hydrogen is replaced by" relates to A¹, A², A³, A³ and A⁵; to R¹ to R⁵; to Ar¹; to L; and to ET; if not otherwise stated.

In the present specification, when a definition is not otherwise provided, "substituted" refers to one substituted with a deuterium, C₁ to C₁₂ alkyl and C₁ to C₁₂ alkoxy.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenylor tolyl, polycyclic groups which comprise more aromatic rings linked by single bonds, like biphenylyl, and polycyclic groups comprising fused rings, like naphtyl or fluoren-2-yl.

Analogously, under heteroaryl, it is understood a group derived by formal abstraction of one ring hydrogen from a heterocyclic aromatic ring in a compound comprising at least one such ring.

Under heterocycloalkyl, it is understood a group derived by formal abstraction of one ring hydrogen from a saturated heterocyclic ring in a compound comprising at least one such ring.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, the single bond refers to a direct bond.

In the context of the present invention, "different" means that the compounds do not have an identical chemical structure.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electron formed in the cathode may be easily injected into the emission layer and transported in the emission layer due to conductive characteristics according to a lowest unoccupied molecular orbital (LUMO) level.

### Advantageous Effects

Surprisingly, it was found that the semiconducting material according to the invention solves the problem underlying the present invention by enabling devices in various aspects superior over the organic electroluminescent devices known in the art, in particular with respect to voltage and/or efficiency. These parameters are important for high efficiency and thereby increased battery life of a mobile device, for example a mobile display device.

The inventors have surprisingly found that particularly good performance can be achieved when using the organic semiconducting material according to the invention as an electron transport layer in a fluorescent blue device.

The specific arrangements mentioned herein as preferred were found to be particularly advantageous.

Further an organic electroluminescent device having high efficiency and/or long life-span may be realized.

Hereinafter, the organic semiconducting material and the device comprising it are described.

### First electron transport matrix compound

Similar as other compounds comprised in the inventive device outside the emitting layer, the first electron transport matrix compound may not emit light under the operation condition of an electroluminescent device, for example an OLED.

According to a further embodiment, the first matrix compound is a compound according to formula (Ia): 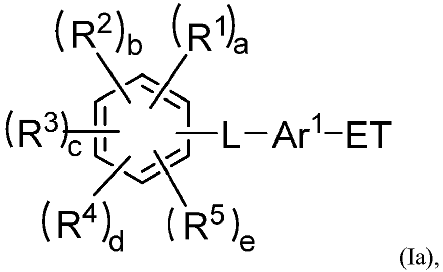 wherein, in formula la,
- Ar¹: is selected from C₆ to C₁₂ aryl and C₁ to C₁₁ heteroaryl;
- R¹ to R⁵: are independently a substituted or unsubstituted C6 to C30 aryl group, a substituted or unsubstituted C₂ to C₃₀ heteroaryl group;
- a to e: are independently an integer of 0 or 1 and 4 ≤ a+b+c+d+e ≤ 5;
- L: is a single bond, a substituted or unsubstituted C₆ to C₃₀ arylene group, or a substituted or unsubstituted C₂ to C₃₀ heteroarylene group;
- ET: is a unsubstituted C₆ to C₄₀ aryl or a unsubstituted C₅ to C₄₀ heteroaryl group, or a substituted C₆ to C₄₀ aryl or a substituted C₅ to C₄₀ heteroaryl group; and
wherein in the substituted group, at least one hydrogen is replaced by
(i) deuterium,
(ii) a halogen,
(iii) a C₂ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(v) a C₁ to C₂₂ silyl group,
(vi) a C₁ to C₃₀ alkyl group,
(vii) a C₁ to C₁₀ alkylsilyl group,
(viii) a C₆ to C₂₂ arylsilyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

In one embodiment, the ET group is not a carbazolyl group.

Formula (Ia) falls under the definition of Formula I, wherein A¹ and A² are a single bond; A³ = L; A⁴ = Ar¹ and A5 = ET.

According to a further embodiment, in formula (Ia):
- R¹ to R⁵: are independently a substituted or unsubstituted C₆ to C₁₂ aryl group, a substituted or unsubstituted C₅ to C₉ heteroaryl group;
- a to e: are independently an integer of 0 or 1 and 4 ≤ a+b+c+d+e ≤ 5;
- L: is a single bond, a substituted or unsubstituted C₆ to C₁₂ arylene group, or a substituted or unsubstituted C₅ to C₉ heteroarylene group;
- ET: is a unsubstituted C₆ to C₁₈ aryl or a unsubstituted C₅ to C₂₀ heteroaryl group or a substituted C₆ to C₁₈ aryl or a substituted C₅ to C₂₀ heteroaryl group; and
wherein in the substituted group, at least one hydrogen is replaced by
(i) deuterium,
(ii) a C₁ to C₁₂ alkyl group,
(iii) a C₆ to C₁₂ aryl group,
(iv) a C₅ to C₉ heteroaryl group, or
(v) a C₁ to C₁₂ alkoxy group.

In one embodiment, the ET group is not a carbazolyl group.

According to a further embodiment, Ar¹ is phenyl or biphenylyl and L is a single bond.

According to a further embodiment, the first electron transport compound is a compound according to formula (Ib): 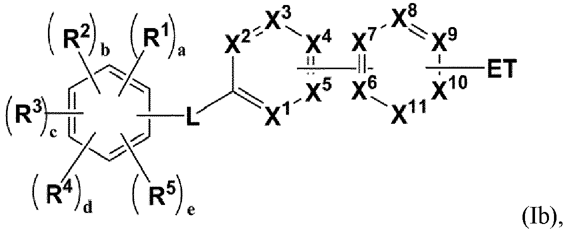 wherein in formula Ib:
- X¹ to X¹¹: are independently, N, C, or CR^{a};
- R^{a}: is independently, hydrogen, deuterium, a C₁ to C₃₀ alkyl group, a C₃ to C₃₀ cycloalkyl group, a C₆ to C₃₀ aryl group, a C₆ to C₃₀ diarylamine group, a C₁ to C₃₀ alkoxy group, a C₃ to C₂₁ silyl group, a C₃ to C₂₁ silyloxy group, a C₁ to C₃₀ alkylthiol group, a C₆ to C₃₀ arylthiol group, a halogen, a C₁ to C₃₀ halogenated hydrocarbyl group, a cyano group;
- R¹ to R⁵: are independently a substituted or unsubstituted C₆ to C₃₀ aryl group, a substituted or unsubstituted C₂ to C₃₀ heteroaryl group;
- a to e: are independently an integer of 0 or 1 and 4 ≤ a+b+c+d+e ≤ 5;
- L: is a single bond, a substituted or unsubstituted C₆ to C₃₀ arylene group, a substituted or unsubstituted C₂ to C₃₀ heteroarylene group;
- ET: is a unsubstituted C₆ to C₄₀ aryl or a unsubstituted C₂ to C₄₀ heteroaryl group, or a substituted C₆ to C₄₀ aryl or a substituted C₂ to C₄₀ heteroaryl group; and
wherein in the substituted group, at least one hydrogen is replaced by
(i) deuterium,
(ii) a halogen,
(iii) a C₁ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(v) a C₁ to C₂₂ silyl group,
(vi) a C₁ to C₃₀ alkyl group,
(vii) a C₁ to C₁₀ alkylsilyl group,
(viii) a C₆ to C₂₂ arylsilyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

Preferably, R^{a} is independently selected from hydrogen, deuterium, a C₁ to C₃₀ alkyl group, a C₃ to C₃₀ cycloalkyl group, a C₆ to C₃₀ aryl group, or a C₁ to C₃₀ alkoxy group. In one embodiment, the ET group is not a carbazolyl group.

According to a further embodiment, the first electron transport compound is a compound according to formula (Ic) 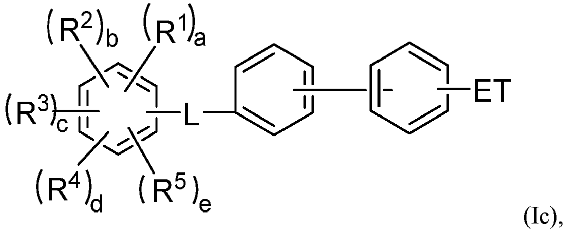 wherein in formula Ic:
- R¹ to R⁵: are independently a substituted or unsubstituted C₆ to C₃₀ aryl group, a substituted or unsubstituted C₂ to C₃₀ heteroaryl group;
- a to e: are independently an integer of 0 or 1 and 4 ≤ a+b+c+d+e ≤ 5,
- L: is a single bond, a substituted or unsubstituted C₆ to C₃₀ arylene group, a substituted or unsubstituted C₂ to C₃₀ heteroarylene group, and
- ET: is a unsubstituted C₆ to C₄₀ aryl or a unsubstituted C₂ to C₄₀ heteroaryl group, or a substituted C₆ to C₄₀ aryl or a substituted C₂ to C₄₀ heteroaryl group; and
wherein in the substituted group, at least one hydrogen is replaced by
(i) deuterium,
(ii) a halogen,
(iii) a C₁ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group
(v) a C₁ to C₂₂ silyl group,
(vi) a C₁ to C₃₀ alkyl group,
(vii) a C₁ to C₁₀ alkylsilyl group,
(viii) a C₆ to C₂₂ arylsilyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

In one embodiment, the ET group is not a carbazolyl group.

According to a further embodiment, in formula (Ic): 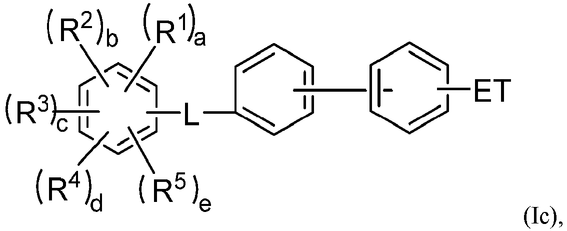
- R¹ to R⁵: are independently a substituted or unsubstituted C₆ to C₃₀ aryl group, a substituted or unsubstituted C₂ to C₃₀ heteroaryl group;
- a to d: are 1;
- e: is 0;
- L: is a single bond, a substituted or unsubstituted C₆ to C₃₀ arylene group, a substituted or unsubstituted C₂ to C₃₀ heteroarylene group,
- ET: is a unsubstituted C₆ to C₄₀ aryl or a unsubstituted C₂ to C₄₀ heteroaryl group, or a substituted C₆ to C₄₀ aryl or a substituted C₂ to C₄₀ heteroaryl group; and
wherein in the substituted group, at least one hydrogen is replaced by
(i) deuterium,
(ii) a halogen,
(iii) a C₁ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₁ to C₂₂ silyl group,
(v) a C₁ to C₃₀ alkyl group,
(vi) a C₁ to C₁₀ alkylsilyl group,
(vii) a C₆ to C₂₂ arylsilyl group,
(viii) a C₃ to C₃₀ cycloalkyl group,
(ix) a C₂ to C₃₀ heterocycloalkyl group,
(x) a C₆ to C₃₀ aryl group,
(xi) a C₂ to C₃₀ heteroaryl group,
(xii) a C₁ to C₂₀ alkoxy group,
(xiii) a C₁ to C₃₀ perfluoro-hydrocarbyl group,
(xiv) a C₁ to C₁₀ trifluoroalkyl group, or
(xv) a cyano group.

According to a further embodiment, in the substituted group one hydrogen atom is replaced by
(i) deuterium,
(ii) a halogen,
(iii) a C₁ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₁ to C₂₂ silyl group,
(v) a C₁ to C₃₀ alkyl group,
(vi) a C₁ to C₁₀ alkylsilyl group,
(vii) a C₆ to C₂₂ arylsilyl group,
(viii) a C₃ to C₃₀ cycloalkyl group,
(ix) a C₂ to C₃₀ heterocycloalkyl group,
(x) a C₆ to C₃₀ aryl group,
(xi) a C₂ to C₃₀ heteroaryl group,
(xii) a C₁ to C₂₀ alkoxy group,
(xiii) a C₁ to C₃₀ perfluoro-hydrocarbyl group,
(xiv) a C₁ to C₁₀ trifluoroalkyl group, or
(xv) a cyano group.

Preferably, R¹ to R⁵ are independently selected from a substituted or unsubstituted C₆ to C₁₈ aryl group or C₅ to C₁₈ heteroaryl group, more preferred from a substituted or unsubstituted C₆ to C₁₈ aryl group. Preferably, R¹ to R⁵ are unsubstituted. In one embodiment, the ET group is not a carbazolyl group.

Particularly good performance can be achieved when the compound of formula I is selected in this range, in particular in layers which are deposited in vacuum.

One or more substituents may be selected from C₄ to C₁₂ alkyl or C₄ to C₁₂ alkoxy.

Particularly good properties in solution processed layers may be obtained, when the compound of formula I is selected in this range.

Preferably, L is selected from a single bond or unsubstituted phenyl.

According to a further embodiment, the ET group is a C₂ to C₃₀ heteroaryl group, preferably ET is selected from formula E1 or E2: 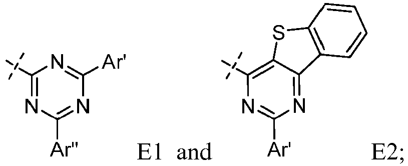 wherein
Ar' and Ar" are independently selected from C₆ to C₁₈ aryl, preferably from C₆ to C₁₂ aryl.

Preferably, ET is selected from formula E1.

Preferably, the compound of formula I is essentially non-emissive.

In the context of the present specification the term "essentially non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one aspect of the invention, compound according to formula (I) may have reduction potential measured by cyclic voltammetry against ferrocene/ferrocenium redox couple, in the range from about - 0.5 V to about - 3.1 V.

According to a further aspect of the invention, the reduction potential of the first electron transport matrix compound, if measured under the same conditions by cyclic voltammetry against Fc/Fc⁺ in tetrahydrofuran, may have a value which is less negative than the value obtained for triphenylphosphine oxide and more negative than the value obtained for tetrakis(quinoxalin-5-yloxy)zirconium.

Under these conditions the reduction potential of triphenylphosphine oxide is about -3.06 V and the reduction potential of tetrakis(quinoxalin-5-yloxy)zirconium is about -1.78 V.

According to a further aspect of the invention, the reduction potential of the first electron transport matrix compound, if measured under the same conditions by cyclic voltammetry against Fc/Fc⁺ in tetrahydrofuran, may have a value which is less negative than the respective value obtained for triphenylphosphine oxide, preferably less negative than the respective value for bis(4-(9H-carbazol-9-yl)phenyl)- (phenyl)phosphine oxide, more preferably less negative than the respective value for 3-([1,1'-biphenyl]-4-yl)-5-(4-(tert-butyl)phenyl)-4-phenyl-4H-1,2,4-triazole, even more preferably less negative than the respective value for pyrene, most preferably less negative than the respective value for 2,7-di-pyrenyl-9,9-spirobifluorene, also preferably less negative than the respective value for 4,7-diphenyl-1,10-phenanthroline, also preferably less negative than the respective value for 2,4,7,9-tetraphenyl-1,10-phenanthroline, also preferably less negative than the respective value for 7-([1,1'-biphenyl]-4-yl)dibenzo[c,h]acridine, also preferably less negative than the respective value for 2,4,6-triphenyltriazine, and still preferably less negative than the respective value for 2,4,6-tri(biphenyl-4-yl)-1,3,5-triazine.

According to a further aspect of the invention, the reduction potential of the first electron transport matrix compound, if measured under the same conditions by cyclic voltammetry against Fc/Fc⁺ in tetrahydrofuran, may have the value which is more negative than the respective value obtained for tetrakis(quinoxalin-5-yloxy)zirconium, preferably more negative than the respective value for 4,4'-bis(4,6-diphenyl-1,3,5-triazin-2-yl)-1,1'-biphenyl, most preferably more negative than the respective value for 2,4,6-tri(biphenyl-4-yl)-1,3,5-triazine.

According to a further aspect of the invention, the reduction potential of the first electron matrix compound may be selected less negative than -2.35 V and more negative than -2.14 V, preferably less negative than -2.3 V and more negative than -2.16 V, more preferably less negative than -2.25 V and more negative than -2.16 V, when measured against Fc/Fc⁺ in tetrahydrofuran.

The reduction potential can be determined by cyclic voltammetry with potentiostatic device Metrohm PGSTAT30 and software Metrohm Autolab GPES at room temperature. The reduction potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard Fc⁺/Fc redox couple, afforded finally the values reported above. All studied compounds as well as the reported comparative compounds showed well-defined reversible electrochemical behaviour.

In one embodiment, the dipole moment of the first matrix compound may be selected ≥ 0 and ≤ 2.3 Debye, preferably ≥ 0.8 and ≤ 2.2 Debye, also preferred ≥ 1 and ≤ 2.2 Debye, also preferred ≥ 1.5 and ≤ 2.2 Debye. In another embodiment, the first matrix compound may have dipole moment higher than 2.3 Debye. It may be a preferred embodiment in combination with redox dopants selected from elemental metals.

According to another aspect, the compound of formula I may have a glass transition temperature (Tg) selected between ≤ 125° C and ≤ 200° C, preferably ≤ 130 ° C and ≤ 180° C.

The glass transition temperature can be measured under nitrogen and using a heating rate of 10 K per min in a Mettler Toledo DSC 822e differential scanning calorimeter as described in DIN EN ISO 11357, published in March 2010.

Particularly preferred may be compounds of formula I with the following structures A1 to A18:

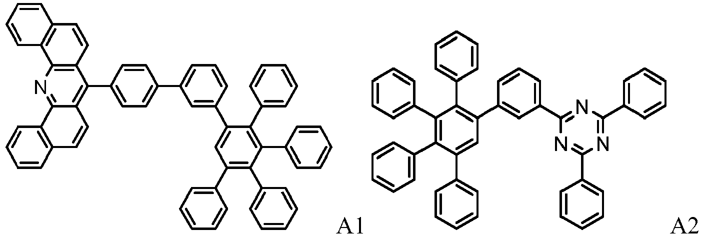

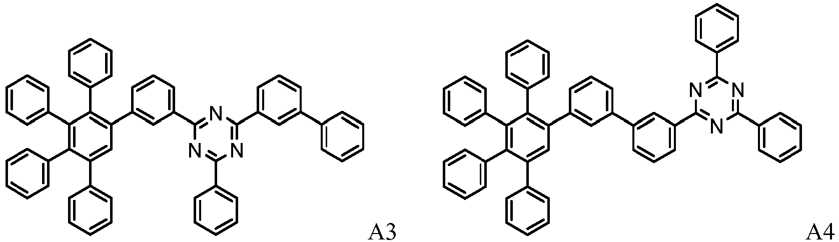

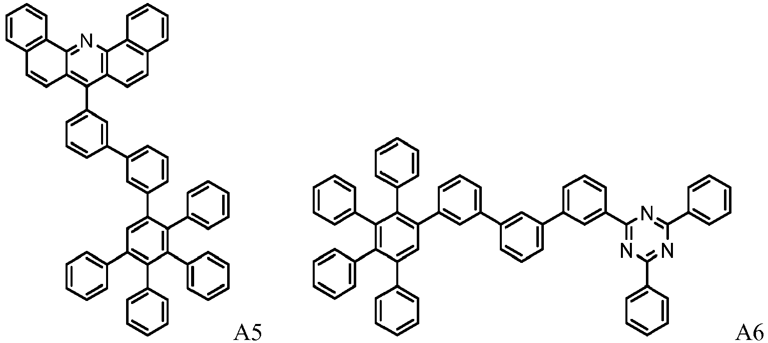

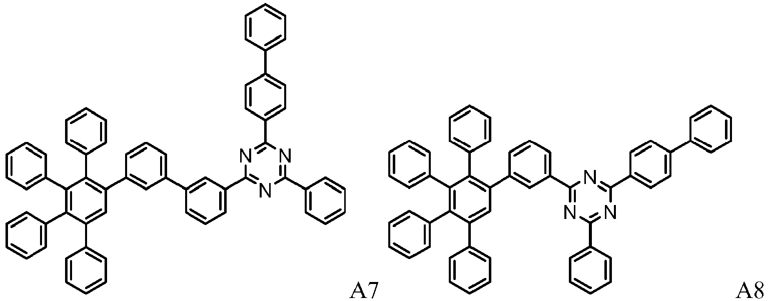

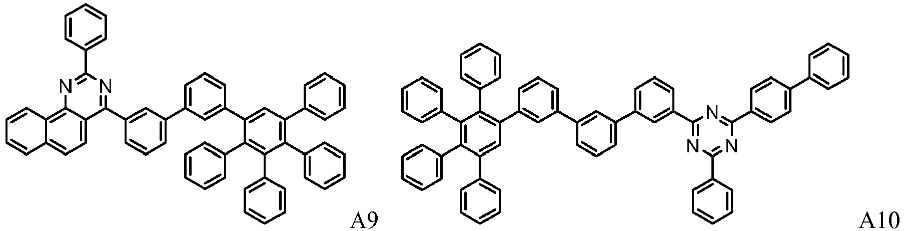

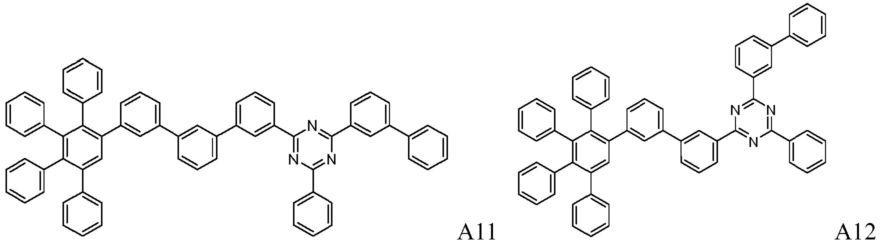

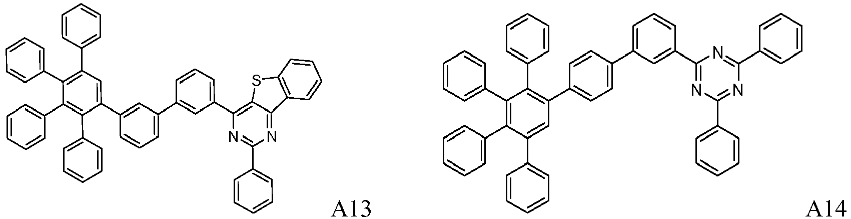

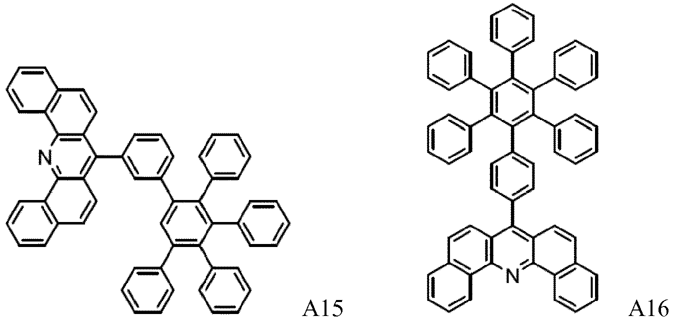

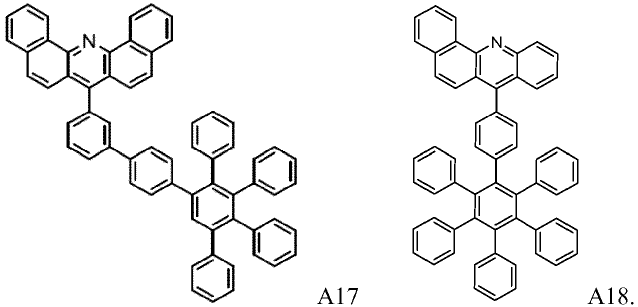

### Electrical n-dopant

Under electrical n-dopant, it is understood a compound which, if embedded into an electron transport matrix, improves, in comparison with the neat matrix under the same physical conditions, the electron properties of the formed semiconducting material, particularly in terms of electron injection and/or electron conductivity.

In the context of the present invention "embedded into an electron transport matrix" means homogenously mixed with the electron transport matrix.

The electrical n-dopant may be selected from elemental metals, metal salts, metal complexes and organic radicals.

In one embodiment, the electrical n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium quinolinolate complex has the formula II, III or IV: 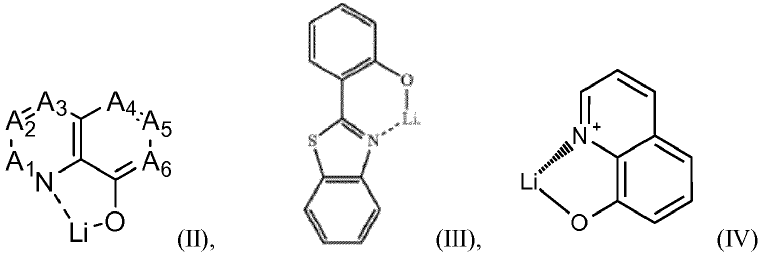 wherein
   A1 to A6 are same or independently selected from CH, CR, N, O;
   R is same or independently selected from hydrogen, halogen, alkyl or aryl or heteroaryl with 1 to 20 carbon atoms; and more preferred A1 to A6 are CH,
- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

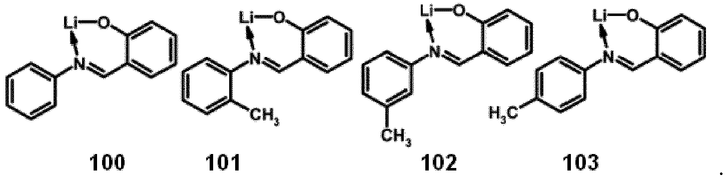

In another embodiment, the electrical n-dopant is a redox n-dopant.

### Redox n-dopant

Under redox n-dopant, it is understood a compound which, if embedded into an electron transport matrix, increases concentration of free electrons in comparison with the neat matrix under the same physical conditions.

The redox n-dopant may not emit light under the operation condition of an electroluminescent device, for example an OLED. In one embodiment, the redox n-dopant is selected from an elemental metal, an electrically neutral metal complex and/or an electrically neutral organic radical.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound. In case that the redox n-dopant is an electrically neutral metal complex and/or an electrically neutral organic radical, the measurement of its redox potential is actually performed for the redox couple formed by
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of
(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.
Electrically neutral metal complexes suitable as redox n-dopants may be e.g. strongly reductive compelxes of some transition metals in low oxidation state. Particularly strong redox n-dopants may be selected for example from Cr(II), Mo(II) and/or W(II) guanidinate complexes such as W₂(hpp)₄, as described in more detail in WO2005/086251.

Electrically neutral organic radicals suitable as redox n-dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form.

It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the n-dopant may be selected from electropositive metals selected from alkali metals, alkaline earth metals, rare earth metals and metals of the first transition period Ti, V, Cr and Mn. Preferably, the n-dopant may be selected from_Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The redox dopant may be essentially non-emissive.

According to another aspect of the invention, it is provided an electronic device comprising a first electrode, a second electrode, and arranged between the first and second electrode, a layer of the organic semiconducting material according to invention. The layer of the semiconducting material according to invention may serve as a charge injection layer or a charge transport layer or a charge generating layer. In one embodiment, the electronic device is an electroluminescent device. Preferably, the electroluminescent device is an organic light emitting diode.

According to another aspect of the invention, it is provided an electronic device comprising at least one electroluminescent device according to any embodiment described throughout this application, preferably, the electronic device comprises the organic light emitting diode in one of embodiments described throughout this application. More preferably, the electronic device is a display device.

### Description of the Drawings

FIG. 1 is a cross-sectional view showing an organic light emitting diode according to an embodiment of the invention.
FIGS. 2 and 3 are cross-sectional views specifically showing a part of an organic layer of an organic light emitting diode according to an embodiment of the invention.

Hereinafter, the figures are illustrated in more detail with reference to examples. However, the present disclosure is not limited to the following figures.

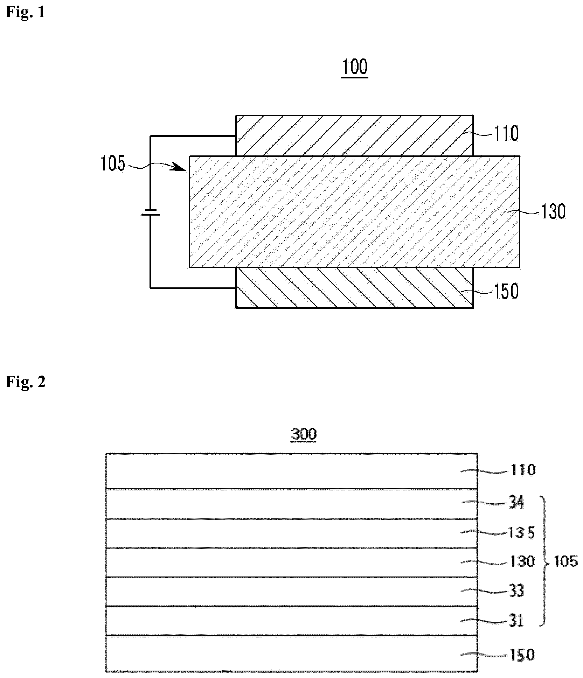

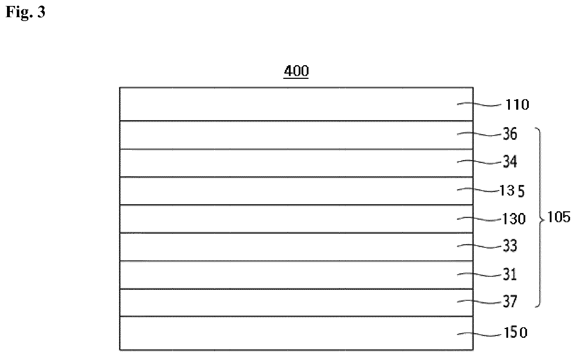

FIGS. 1 to 3 are schematic cross-sectional views of organic light emitting diodes 100, 300, and 400 according to an embodiment of the present invention. Hereinafter, referring to FIG 1, a structure of an organic light emitting diode according to an embodiment of the present invention and a method of manufacturing the same are as follows. The organic light emitting diode 100 has a structure where an anode 110, a stack of organic layers 105 including an optional hole transport region; an emission layer 130; and a cathode 150 that are sequentially stacked.

A substrate may be disposed on the anode 110 or under the cathode 150. The substrate may be selected from usual substrate used in a general organic light emitting diode and may be a glass substrate or a transparent plastic substrate.

The anode 110 may be formed by depositing or sputtering an anode material on a substrate. The anode material may be selected from materials having a high work function that makes hole injection easy. The anode 110 may be a reflective electrode, a transflective electrode, or a transmissive electrode. The anode material may use indium tin oxide (ITO), indium zinc oxide (IZO), tin oxide (SnO₂), zinc oxide (ZnO), and the like. Or, it may be a metal such as silver (Ag), or gold (Au), or an alloy thereof.

The anode 110 may have a monolayer or a multi-layer structure of two or more layers.

The organic light emitting diodes 100, 300, and 400 according to an embodiment of the present invention may include a hole transport region; an emission layer 130; and a first electron transport layer 31 comprising a compound according to formula I.

Referring to FIG. 2, the hole transport region of the stack of organic layers 105 may include at least two layered hole transport layers, and in this case, the hole transport layer contacting the emission layer (130) is defined as a second hole transport layer 135 and a the hole transport layer contacting the anode (110) is defined as a first hole transport layer 34. The stack of organic layers 105 further includes two electron transport layers, namely second electron transport layer 33 and the first electron transport layer 31. The hole transport region of the stack 105 may further include at least one of a hole injection layer, a hole transport layer, an electron blocking layer, and a buffer layer.

The hole transport region of the stack 105 may include only hole injection layer or only hole transport layer. Or, the hole transport region may have a structure where a hole injection layer 36/hole transport layer 34 or hole injection layer 36/hole transport layer 34/electron blocking layer (135) is sequentially stacked from the anode 110.

For example, the hole injection layer 36 and the electron injection layer 37 can be additionally included, so that an OLED may comprise an anode 110/hole injection layer 36/first hole transport layer 34/electron blocking layer 135/emission layer 130/second electron transport layer 33/ first electron transport layer 31/electron injection layer 37/cathode 150, which are sequentially stacked.

According to another aspect of the invention, the organic electroluminescent device (400) comprises an anode (110), a hole injection layer (36), a first hole transport layer (34), optional an electron blocking layer (135), an emission layer (130), second electron transport layer (33), first electron transport layer (31), an optional electron injection layer (37), a cathode (150) wherein the layers are arranged in that order.

The hole injection layer 36 may improve interface properties between ITO as an anode and an organic material used for the hole transport layer 34, and is applied on a non-planarized ITO and thus planarizes the surface of the ITO. For example, the hole injection layer 36 may include a material having a median value of the energy level of its highest occupied molecular orbital (HOMO) between the work function of ITO and the energy level of the HOMO of the hole transport layer 34, in order to adjust a difference between the work function of ITO as an anode and the energy level of the HOMO of the first hole transport layer 34.

When the hole transport region includes a hole injection layer 36, the hole injection layer may be formed on the anode 110 by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of about 100 °C to about 500 °C, a pressure of about 10⁻⁶ Pa to about 10⁻¹ Pa, and a deposition rate of about 0.1 to about 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of about 2000 rpm to about 5000 rpm, and a temperature at which heat treatment is performed to remove a solvent after coating may be in a range of about 80 °C to about 200 °C, but the coating conditions are not limited thereto.

Conditions for forming the hole transport layer and the electron blocking layer may be defined based on the above-described formation conditions for the hole injection layer.

A thickness of the hole transport part of the charge transport region may be from about 10 nm to about 1000 nm, for example, about 10 nm to about 100 nm. When the hole transport transport part of the charge transport region includes the hole injection layer and the hole transport layer, a thickness of the hole injection layer may be from about 10 nm to about 1000 nm, for example about 10 nm to about 100 nm and a thickness of the hole transport layer may be from about 5 nm to about 200 nm, for example about 10 nm to about 150 nm. When the thicknesses of the hole transport part of the charge transport region, the HIL, and the HTL are within these ranges, satisfactory hole transport characteristics may be obtained without a substantial increase in driving voltage.

Hole transport matrix materials used in the hole transport region are not particularly limited. Preferred are covalent compounds comprising a conjugated system of at least 6 delocalized electrons. The term "covalent compound" is in more detail explained below, in the paragraph regarding the second electron transport matrix. Typical examples of hole transport matrix materials which are widely used in hole transport layers are polycyclic aromatic hydrocarbons, triaryl amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

The hole transport region of the stack of organic layers may further include a charge-generating material to improve conductivity, in addition to the materials as described above. The charge-generating material may be homogeneously or non-homogeneously dispersed in the hole transport region.

The charge-generating material may be, for example, a p-dopant. The p-dopant may be one of a quinone derivative, a metal oxide, and a cyano group-containing compound, but is not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), and the like; metal oxides such as tungsten oxide, molybdenum oxide, and the like; and cyano-containing compounds such as compound HT-D1 below.

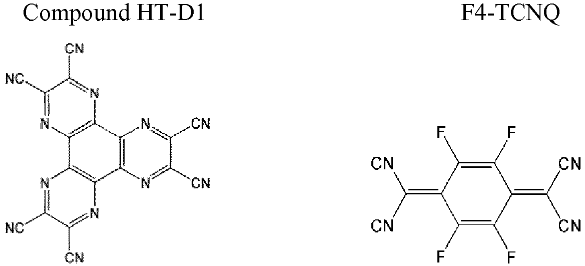

The hole transport part of the charge transport region may further include a buffer layer.

The buffer layer may compensate for an optical resonance distance of light according to a wavelength of the light emitted from the EML, and thus may increase efficiency.

The emission layer (EML) may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

The emitter may be a red, green, or blue emitter.

In one embodiment, the emitter host is an anthracene matrix compound represented by formula 400 below:

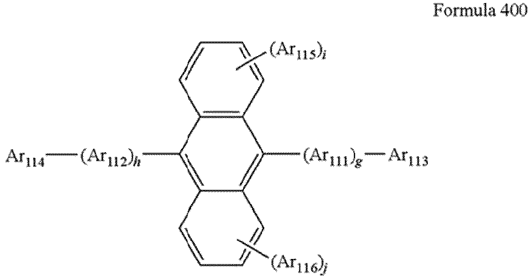

In formula 400, Ar₁₁₁ and Ar₁₁₂ may be each independently a substituted or unsubstituted C₆-C₆₀ arylene group; Ar₁₁₃ to Ar₁₁₆ may be each independently a substituted or unsubstituted C₁-C₁₀ alkyl group or a substituted or unsubstituted C₆-C₆₀ aryl group; and g, h, i, and j may be each independently an integer from 0 to 4. In some embodiments, Ar₁₁₁ and Ar₁₁₂ in formula 400 may be each independently one of a phenylene group, a naphthylene group, a phenanthrenylene group, or a pyrenylene group; or a phenylene group, a naphthylene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2. In formula 400, Ar₁₁₃ to Ar₁₁₆ may be each independently one of
- a C₁-C₁₀ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or
- a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof,
- a phosphoric acid group or a salt thereof,
- a C₁-C₆₀ alkyl group, a C₂-C₆₀ alkenyl group, a C₂-C₆₀ alkynyl group, a C₁-C₆₀ alkoxy group, a phenyl group, a naphthyl group, an anthryl group, a pyrenyl group, a phenanthrenyl group, or
- a fluorenyl group; or 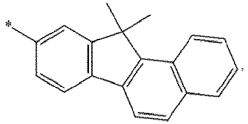 or formulas (Y2) or (Y3):

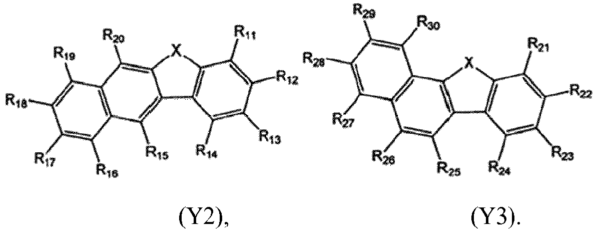

Wherein in the formulas (Y2) and (Y3), X is selected form an oxygen atom and a sulfur atom, but embodiments of the invention are not limited thereto.

In the formula (Y2), any one of R₁₁ to R₁₄ is used for bonding to Ar₁₁₁. R₁₁ to R₁₄ that are not used for bonding to Ar₁₁₁ and R₁₅ to R₂₀ are the same as R₁ to R₈.

In the formula (Y3), any one of R₂₁ to R₂₄ is used for bonding to Ar₁₁₁. R₂₁ to R₂₄ that are not used for bonding to Ar₁₁₁ and R₂₅ to R₃₀ are the same as R₁ to R₈.

Preferably, the EML host comprises between one and three heteroatoms selected from the group consisting of N, O or S. More preferred the EML host comprises one heteroatom selected from S or O.

According to a further aspect of the invention, the emitter host respectively has a reduction potential which, if measured under the same conditions by cyclic voltammetry against Fc/Fc⁺ in tetrahydrofuran, has a value more negative than the respective value obtained for 7-([1,1'-biphenyl]-4-yl)dibenzo[c,h]acridine, preferably more negative than the respective value for 9,9',10,10'-tetraphenyl-2,2'-bianthracene, more preferably more negative than the respective value for 2,9-di([1,1'-biphenyl]-4-yl)-4,7-diphenyl-1,10-phenanthroline, even more preferably more negative than the respective value for 2,4,7,9-tetraphenyl-1,10-phenanthroline, even more preferably more negative than the respective value for 9,10-di(naphthalen-2-yl)-2-phenylanthracene, even more preferably more negative than the respective value for 2,9-bis(2-methoxyphenyl)-4,7-diphenyl-1,10-phenanthroline, most preferably more negative than the respective value for 9,9'-spirobi[fluorene]-2,7-diylbis(diphenylphosphine oxide).

The emitter is mixed in a small amount to cause light emission, and may be generally a material such as a metal complex that emits light by multiple excitation into a triplet or more. The emitter may be, for example an inorganic, organic, or organometallic compound, and one or more kinds thereof may be used.

The emitter may be a fluorescent emitter, for example ter-fluorene, the structures are shown below. 4.4'-bis(4-diphenyl amiostyryl)biphenyl (DPAVBi), 2,5,8,11-tetra-tert-butyl perylene (TBPe), and Compound 4 below are examples of fluorescent blue emitters.

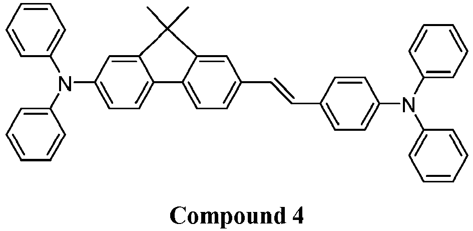

According to another aspect, the organic semiconductor layer comprising a compound of formula I is arranged between a fluorescent blue emission layer and the cathode electrode.

The emitter may be a phosphorescent emitter, and examples of the phosphorescent emitters may be organometallic compounds including Ir, Pt, Os, Ti, Zr, Hf, Eu, Tb, Tm, Fe, Co, Ni, Ru, Rh, Pd, or a combination thereof. The phosphorescent emitter may be, for example a compound represented by formula Z, but is not limited thereto:

L₂MX (Z).

In formula Z, M is a metal, and L and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or, in a polynuclear complex,a combination thereof, and the L and X may be, for example, a bidendate ligand.

A thickness of the emission layer may be about 10 nm to about 100 nm, for example about 20 nm to about 60 nm. When the thickness of the emission layer is within these ranges, the emission layer may have improved emission characteristics without a substantial increase in a driving voltage.

Next, the electron transport region of the stack of organic layers 105 is disposed on the emission layer.

The electron transport region of the stack of organic layers includes at least the first electron transport layer. The electron transport region of the stack of organic layers may further include an electron injection layer and/or the second electron transport layer. At least the first electron transport layer comprises the n-doped semiconducting material according to one of its various embodiments.

For example, the electron transport region of the stack of organic layers may have a structure of the first electron transport layer/second electron transport layer/electron injection layer but is not limited thereto. For example, an organic light emitting diode according to an embodiment of the present invention includes at least two electron transport layers in the electron transport region of the stack of organic layers 105, and in this case, the electron transport layer contacting the emission layer is defined as the second electron transport layer 33.

The electron transport layer may include two or more different electron transport matrix compounds.

### Second electron transport matrix compound

Various embodiments of the electron transport region in the device according to invention, e.g. devices comprising hole blocking layers, electron injecting layers, may comprise a second electron transport matrix compound.

Second electron transport matrix compound is not particularly limited. Similarly as other materials which are in the inventive device comprised outside the emitting layer, the second electron transport matrix compound may not emit light.

According to one embodiment, the second electron transport matrix can be an organic compound, an organometallic compound, or a metal complex.

According to one embodiment, the second electron transport matrix may be a covalent compound comprising a conjugated system of at least 6 delocalized electrons. Under a covalent material in a broadest possible sense, it might be understood a material, wherein at least 50 % of all chemical bonds are covalent bonds, wherein coordination bonds are also considered as covalent bonds. In the present application, the term encompasses in the broadest sense all usual electron transport matrices which are predominantly selected from organic compounds but also e.g. from compounds comprising structural moieties which do not comprise carbon, for example substituted 2,4,6-tribora-1,3,5 triazines, or from metal complexes, for example aluminium tris(8-hydroxyquinolinolate).

The molecular covalent materials can comprise low molecular weight compounds which may be, preferably, stable enough to be processable by vacuum thermal evaporation (VTE). Alternatively, covalent materials can comprise polymeric covalent compounds, preferably, compounds soluble in a solvent and thus processable in form of a solution. It is to be understood that a polymeric substantially covalent material may be crosslinked to form an infinite irregular network, however, it is supposed that such crosslinked polymeric substantially covalent matrix compound still comprises both skeletal as well as peripheral atoms. Skeletal atoms of the covalent compound are covalently bound to at least two neighbour atoms. Other atoms of the covalent compound are peripheral atoms which are covalently bound with a single neighbour atom. Inorganic infinite crystals or fully crosslinked networks having partly covalent bonding but substantially lacking peripheral atoms, like silicon, germanium, gallium arsenide, indium phosphide, zinc sulfide, silicate glass etc. are not considered as covalent matrices in the sense of present application, because such fully crosslinked covalent materials comprise peripheral atoms only on the surface of the phase formed by such material. A compound comprising cations and anions is still considered as covalent, if at least the cation or at least the anion comprises at least ten covalently bound atoms.

Preferred examples of covalent second electron transport matrix compounds are organic compounds, consisting predominantly from covalently bound C, H, O, N, S, which may optionally comprise also covalently bound B, P, As, Se. In one embodiment, the second electron transport matrix compound lacks metal atoms and majority of its skeletal atoms is selected from C, O, S, N.

In another embodiment, the second electron transport matrix compound comprises a conjugated system of at least six, more preferably at least ten, even more preferably at least fourteen delocalized electrons.

Examples of conjugated systems of delocalized electrons are systems of alternating pi- and sigma bonds. Optionally, one or more two-atom structural units having the pi-bond between its atoms can be replaced by an atom bearing at least one lone electron pair, typically by a divalent atom selected from O, S, Se, Te or by a trivalent atom selected from N, P, As, Sb, Bi. Preferably, the conjugated system of delocalized electrons comprises at least one aromatic or heteroaromatic ring adhering to the Hückel rule. Also preferably, the second electron transport matrix compound may comprise at least two aromatic or heteroaromatic rings which are either linked by a covalent bond or condensed.

In one of specific embodiments, the second electron transport matrix compound comprises a ring consisting of covalently bound atoms and at least one atom in the ring is phosphorus.

In a more preferred embodiment, the phosphorus-containing ring consisting of covalently bound atoms is a phosphepine ring.

In another preferred embodiment, the covalent matrix compound comprises a phosphine oxide group. Also preferably, the substantially covalent matrix compound comprises a heterocyclic ring comprising at least one nitrogen atom. Examples of nitrogen containing heterocyclic compounds which are particularly advantageous as second electron transport matrix compound for the inventive device are matrices comprising, alone or in combination, pyridine structural moieties, diazine structural moieties, triazine structural moieties, quinoline structural moieties, benzoquinoline structural moieties, quinazoline structural moieties, acridine structural moieties, benzacridine structural moieties, dibenzacridine structural moieties, diazole structural moieties and benzodiazole structural moieties.

The second matrix compound may have a molecular weight (Mw) of ≥ 400 to ≤ 850 g / mol, preferably ≥ 450 to ≤ 830 g / mol. If the molecular weight is selected in this range, particularly reproducible evaporation and deposition can be achieved in vacuum at temperatures where good long-term stability is observed.

Preferably, the second matrix compound may be essentially non-emissive.

According to another aspect, the reduction potential of the second electron transport compound may be selected more negative than -2.2 V and less negative than -2.35 V against Fc/Fc⁺ in tetrahydrofuran, preferably more negative than -2.25 V and less negative than -2.3 V.

According to one embodiment, the first and the second matrix compound may be selected different, and
- the second electron transport layer consist of a second matrix compound; and
- the first electron transport layer consist of the first matrix compound of formula (I), and an electrical n-dopant, preferably an alkali metal salt or an alkali metal organic complex.

Preferably, the first and second electron transport layer may be essentially non-emissive.

According to another embodiment, the second electron transport layer can be in direct contact with the emission layer.

According to another embodiment, the first electron transport layer can be in direct contact with the second electron transport layer.

According to another embodiment, the second electron transport layer can be contacting sandwiched between the emission layer and the first electron transport layer.

According to another embodiment, the first electron transport layer can be in direct contact with the electron injection layer.

According to another embodiment, the first electron transport layer can be contacting sandwiched between the second electron transport layer and the electron injection layer.

According to another embodiment, the first electron transport layer can be in direct contact with the cathode electrode.

According to another embodiment, the first electron transport layer can be contacting sandwiched between the second electron transport layer and the cathode layer.

According to another embodiment, the second electron transport layer can be contacting sandwiched between the emission layer and the first electron transport layer, and the first electron transport layer can be contacting sandwiched between the second electron transport layer and the electron injection layer.

The formation conditions of the first electron transport layer 31, second electron transport layer 33, and electron injection layer 37 of the electron transport region of the stack of organic layers refer to the formation conditions of the hole injection layer.

The thickness of the first electron transport layer may be from about 2 nm to about 100 nm, for example about 3 nm to about 30 nm. When the thickness of the first electron transport layer is within these ranges, the first electron transport layer may have improved electron transport auxiliary ability without a substantial increase in driving voltage.

A thickness of the second electron transport layer may be about 10 nm to about 100 nm, for example about 15 nm to about 50 nm. When the thickness of the electron transport layer is within these ranges, the electron transport layer may have satisfactory electron transporting ability without a substantial increase in driving voltage.

According to another aspect of the invention, the organic electroluminescent device further comprises an electron injection layer between the second electron transport layer and the cathode.

The electron injection layer (EIL) 37 may facilitate injection of electrons from the cathode 150.

According to another aspect of the invention, the electron injection layer 37 comprises:
(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt, more preferably a Li quinolinolate, even more preferably a lithium 8-hydroxyquinolinolate, most preferably the alkali metal salt and/or complex of the second electron transport layer is idencial with the alkali metal salt and/or complex of the injection layer.

The electron injection layer may include at least one selected from LiF, NaCl, CsF, Li₂O, and BaO.

A thickness of the EIL may be from about 0.1 nm to about 10 nm, or about 0.3 nm to about 9 nm. When the thickness of the electron injection layer is within these ranges, the electron injection layer may have satisfactory electron injection ability without a substantial increase in driving voltage.

A material for the cathode 150 may be a metal, an alloy, or an electrically conductive compound that have a low work function, or a combination thereof. Specific examples of the material for the cathode 150 may be lithium (Li), magnesium (Mg), aluminum (Al), aluminum-lithium (Al-Li), calcium (Ca), magnesium-indium (Mg-In), magnesium-silver (Mg-Ag), etc. In order to manufacture a top-emission light-emitting device having a reflective anode 110 deposited on a substrate, the cathode 150 may be formed as a transmissive electrode from, for example, indium tin oxide (ITO) or indium zinc oxide (IZO).

Hereinafter, the embodiments are illustrated in more detail with reference to examples. However, the present disclosure is not limited to the following examples.

### Detailed description

### Synthesis and physical properties of compound of formula I

Triazine compounds of formula I may be synthesized in accordance with the methods described in PCT-KR2015-012551.

### Synthesis Example 1: Compound A6 (in the scheme referred as compound_[3])

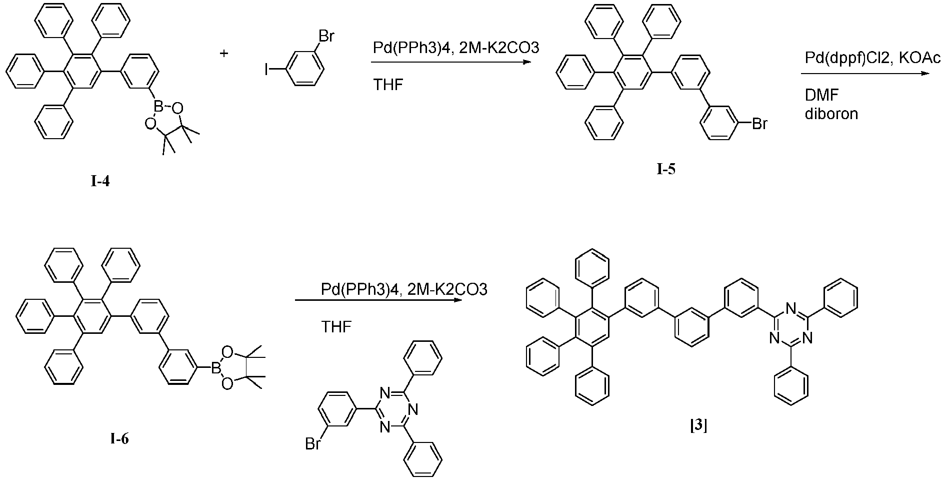

### First Step: Synthesis of Intermediate I-5

13 g of an intermediate I-5 (61 %) was obtained in the same synthesis method as the synthesis method of the compound 1 by using the intermediate I-4 (20.4 g, 34.92 mmol) and 1-bromo-3-iodobenzene (16.5 g, 52.39 mmol) under a nitrogen environment.

### Second Step: Synthesis of Intermediate I-6

10 g of an intermediate I-6 (74 %) was obtained in the same synthesis method as the synthesis method of the intermediate I-4 by using the intermediate I-5 (12.6 g, 20.54 mmol) under a nitrogen environment.

### Third Step: Synthesis of Compound A6

8.7 g of compound A6 (in the scheme referred as [3]) was obtained in 68 % yield by using the intermediate I-6 (10 g, 15.2 mmol) and 2-(3-bromophenyl)-4,6-diphenyl-1,3,5-triazine (7.9 g, 18.32 mmol). These reagents were dissolved in 250 mL tetrahydrofuran under a nitrogen environment, tetrakis(triphenylphosphine)palladium (0.9 g, 0.75 mmol) was added thereto, and the mixture was stirred. Then, potassium carbonate saturated in water (5.2 g, 37 mmol) was added thereto, and the mixture was heated and refluxed at 80°C for 24 hours. When the reaction was complete, water was added to the reaction solution, dichloromethane was used to perform an extraction, an anhydrous MgSO₄ was used to remove moisture therefrom, and a resultant therefrom was filtered and concentrated under a reduced pressure. This obtained residue was separated and purified through column chromatography.
LC Mass (theoretical value: 842.04 g/mol, measured value: M+H⁺ = 843.03 g/mol)

The benzoquinazoline compound A9 was prepared analogously. Physical properties of tested compounds of formula (I) are summarized in Table 1.

Dibenzoacridine compounds of formula I may be synthesized in accordance with the methods described in WO2011/154131A1.

Another alternative is demonstrated in Synthesis example 2. The procedure is generally applicable for the synthesis of compounds comprising the hexaphenylbenzene structural moiety.

### Synthesis example 2: compound A16

### Step 1: Synthesis of 7-(4-(phenylethynyl)phenyl)dibenzo[c,h]acridine

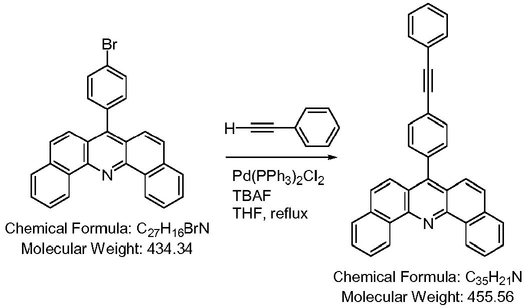

A three necked 250-mL round bottom flask is purged with N₂. Under a constant flow of N₂ 7-(4-bromophenyl)dibenzo[*c,h*]acridine (10.0 g, 23.0 mmol), phenylacetylene (4.70 g, 46.0 mmol, 2.0 eq.), and bis (triphenylphosphine)-palladium chloride (3.23 g, 4.6 mmol, 0.2 eq.) were introduced, followed by a 1M-solution of tetrabutylammonium fluoride in THF (70 mL). The resulting mixture was warmed up to reflux and reacted for 2h. After completion of the reaction, MeOH (70 mL) was added, and the solution was left to cool down to room temperature. The precipitate formed upon cooling was collected by filtration, washed with MeOH (2 x 50 mL), then hexane (3 x 50 mL), and finally dried under vacuum at 40°C.
Yield: about 7.0 g (about 67%, yellowish solid).

### Step 2: Synthesis of 7-(3',4',5',6'-tetraphenyl-[1,1':2',1"-terphenyl]-4-yl)dibenzo[c,h]acridine

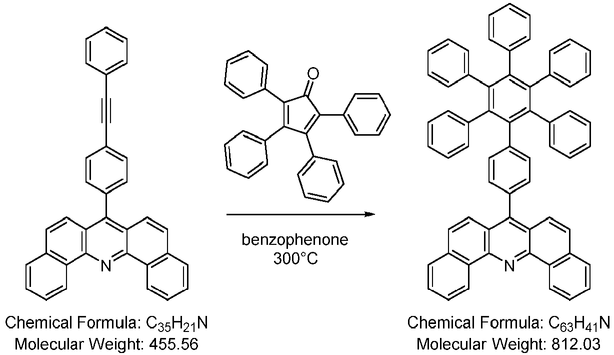

A three necked 100-mL round bottom flask was charged with 7-(4-(phenylethynyl)phenyl)dibenzo[*c,h*]acridine (6.8 g, 14.9 mmol), 2,3,4,5-tetraphenylcyclopenta-2,4-dienone (6.31 g, 16.4 mmol, 1.1 eq.), and benzophenone (35 g as molten solvent). After degassing the solids with N₂, the resulting mixture was warmed up to 300°C. After 1h of reflux at 300°C, gas evolution had stopped and the mixture was hence cooled down to *ca.* 80°C. Toluene (100 mL), was added, and the resulting precipitate was filtered off and washed with toluene (2 x 40 mL), followed by hexane (2 x 40 mL). The solid was then purified by trituration in hot chlorobenzene (60 mL), followed by trituration in hot MeOH (60 mL). After filtration and drying under vacuum at 120°C, the desired was isolated as a yellowish powder.
Yield: about 6.8 g (about 56%, yellowish solid).

The benzoacridine compound A18 was prepared analogously. In Table 1 are summarized dibenzoacridine compounds of formula I and their starting material, yield, m/z, glass transition temperature, reduction potential against Fc/Fc⁺ in tetrahydrofuran.

**Table 1**

| Comp. I: | Starting materials | Structure of compound I | Yield [%] | Tg [°C] | Redox potential against Fc/Fc⁺ [V] |
|---|---|---|---|---|---|
| A1 | 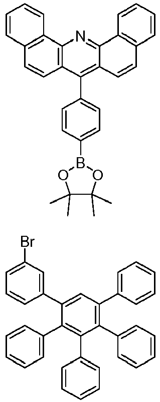 | 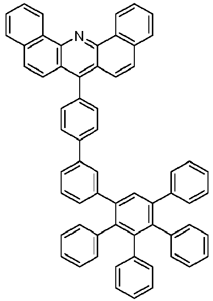 | 62% | 175 | -2.25 |
| A2 | | 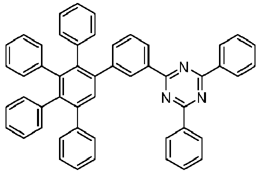 | | 138 | -2.20 |
| A3 | | 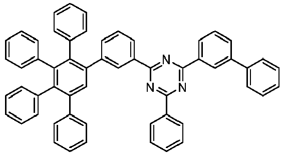 | | 135 | -2.20 |
| A4 | | 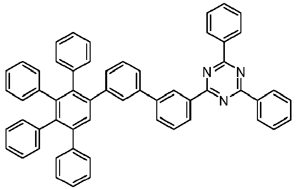 | | 140 | -2.22 |
| A5 | 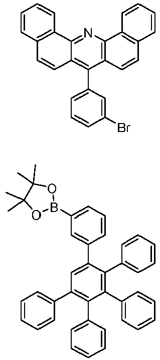 | 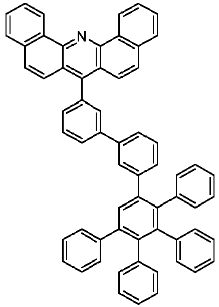 | 86% | 165 | -2.29 |
| A6 | | 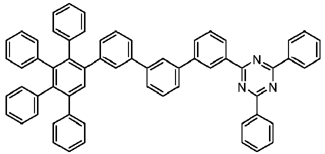 | | 139 | -2.18 |
| A7 | | 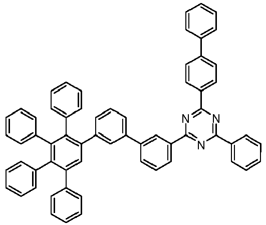 | | 147 | -2.15 |
| A8 | | 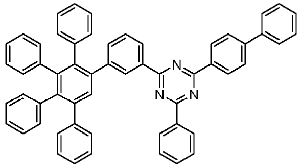 | | 147 | -2.18 |
| A9 | | 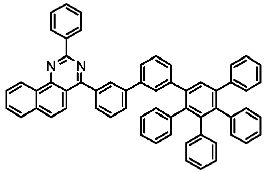 | | 144 | -2.25 |
| A10 | | 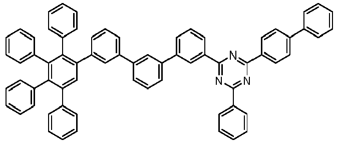 | | 149 | -2.14 |
| A12 | | 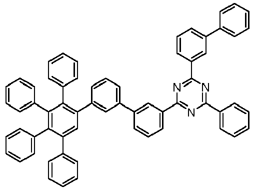 | | - | -2.18 |
| A13 | | | | - | -2.23 |
| A15 | | | 58% | 159 | -2.29 |
| A16 | | 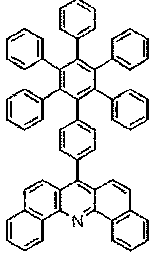 | | Not observed | -2.31 |
| A17 | | | 50% | 175 | - |
| A18 | | 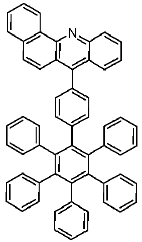 | | Not observed | -2.25 |

### General procedure for fabrication of OLEDs

The model top emitting blue fluorescent OLED is described below. It was prepared using auxiliary materials F1, F2, F3, F4, F5, F6 and PD-2: 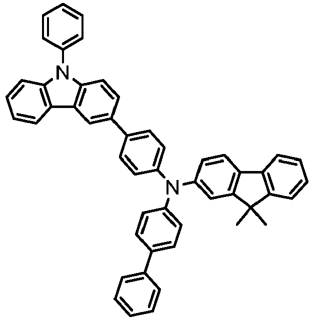 biphenyl-4-yl(9,9-dimethyl-9H-fluoren-2-yl)-[4-(9-phenyl-9H-carbazol-3-yl)phenyl]-amine, CAS 1242056-42-3, F1; 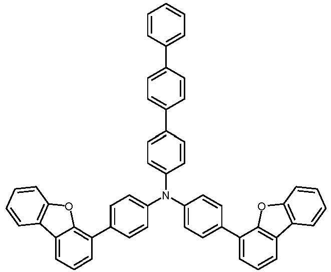 N,N-bis(4-(dibenzo[b,d]furan-4-yl)phenyl)-[1,1':4',1"-terphenyl]-4-amine, CAS 1198399-61-9, F2; 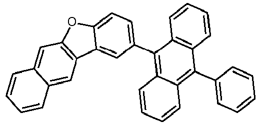 2-(10-phenyl-9-anthracenyl)-benzo[b]naphtho[2,3-d]furan, CAS 1627916-48-6, F3; 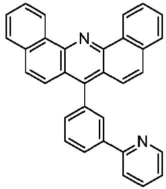 7-(3-(pyridine-2-yl)phenyl)dibenzo[c,h]acridine, F4 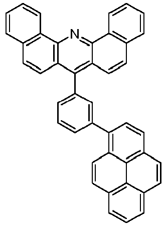 7-(3-(pyren-1-yl)phenyl)dibenzo[c,h]acridine, F5 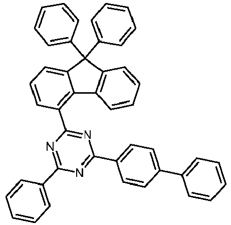 2-([1,1'-biphenyl]-4-yl)-4-(9,9-diphenyl-9H-fluoren-4-yl)-6-phenyl-1,3,5-triazine, CAS 1801992-44-8, F6 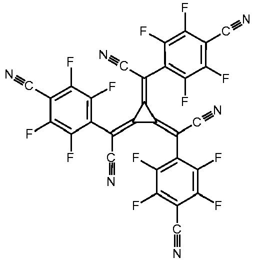 2,2',2"-(cyclopropane-1,2,3-triylidene)tris(2-(p-cyanotetrafluorophenyl)acetonitrile), CAS 1224447-88-4, PD-2.

### Device example 1 (top emitting blue OLED)

A glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited as anode at a pressure of 10⁻⁵ to 10⁻⁷ mbar.

Then, 92 wt.-% F1 with 8 wt.-% PD2 were vacuum deposited on the ITO electrode, to form a HIL having a thickness of 10 nm. Then, undoped F1 was vacuum deposited on the HIL, to form a HTL having a thickness of 122 nm.
Then, F2 was vacuum deposited on the HTL, to form an electron blocking layer (EBL) having a thickness of 5 nm.

Then, 97 wt.-% F3 as EML host and 3 wt.-% blue dopant NUBD370 (Sun Fine Chemicals) were deposited on the EBL, to form a blue-emitting EML with a thickness of 20 nm.

Then the second electron transport layer 33, if present, is formed with a thickness of 5 nm by depositing compound A6, and the first electron transport layer 31 is formed either directly on the emission layer or on the second electron transport layer according. If the first electron transport layer is in direct contact with the emission layer, the thickness is 36 nm. If the first electron transport layer is deposited on top of the second electron transport layer, the thickness is 31 nm.

The first electron transport layer comprises 50 wt.-% matrix compound and 50 wt.-% of

LiQ. The composition is shown in Table 2.

Then the electron injection layer 37 is formed on the electron transport layer 31 by depositing LiQ with a thickness of 1.5 nm or Yb with a thickness of 2 nm.

The cathode was evaporated at ultra-high vacuum of 10⁻⁷ mbar. Therefore, a thermal single co-evaporation of one or several metals was performed with a rate of 0, 1 to 10 nm/s (0.01 to 1 Å/s) in order to generate a homogeneous cathode with a thickness of 5 to 1000 nm. The cathode was formed from 13 nm magnesium silver alloy (90:10 vol.-%) or from 11 nm Ag.

A cap layer of F1 was formed on the cathode with a thickness of 60 nm in case of MgAg cathode and 75 nm in case of Ag cathode.

### Evaluation of device experiments

To assess the performance of the inventive examples compared to the prior art, the current efficiency is measured under ambient conditions (20°C). Operational voltage measurements are performed using a Keithley 2400 sourcemeter, and reported in V at standard current density 10 mA/cm² for top emission devices. For bottom emission devices, the standard current density is usually 15 mA/cm². A calibrated spectrometer CAS140 from Instrument Systems is used for measurement of CIE coordinates and brightness in Candela. Lifetime LT of the device is measured at ambient conditions (20°C) and standard current density 10 mA/cm² or 15 mA/cm², using a Keithley 2400 sourcemeter, and recorded in hours. The brightness of the device is measured using a calibrated photo diode. The lifetime LT is defined as the time till the brightness of the device is reduced to 97 % of its initial value.

The light output in external efficiency EQE and power efficiency P_{eff} (lm/W) are determined at 10 mA/cm² for top emission devices.

To determine the efficiency EQE in % the light output of the device is measured using a calibrated photodiode.

To determine the power efficiency in lm/W, in a first step the luminance in candela per square meter (cd/m²) is measured with an array spectrometer CAS 140 CT from Instrument Systems which has been calibrated by Deutsche Akkreditierungsstelle (DAkkS). In a second step, the luminance is then multiplied by π and divided by the voltage and current density.

In bottom emission devices, the emission is predominately Lambertian and quantified in percent external quantum efficiency (EQE) and power efficiency in lm/W. The auxiliary compounds F4-F6 served as state-of-art references; the results in terms of operational voltage U, and current efficiency C_{eff} are shown in Table 2.

**Table 2: Performance at 10 mA/cm² of top emission devices comprising a second ETL (33), a first ETL (34) and a lithium organic complex, and an EIL (37)**

| | second ETL | first ETL | EIL | Cathode | U (V) | C_{eff} (cd/A) |
|---|---|---|---|---|---|---|
| Comparative device 1 | - | F4:LiQ | LiQ | Mg:Ag | 3.39 | 7.2 |
| Device 1 | - | A15:LiQ | Yb | Ag | 3.71 | **9.2** |
| Device 2 | - | A5:LiQ | Yb | Ag | 3.56 | **9.2** |
| Comparative device 2 | A6 | F5:LiQ | LiQ | Mg:Ag | 3.41 | 6.5 |
| Device 3 | A6 | A16:LiQ | Yb | Ag | 3.77 | **9.2** |
| Device 4 | A6 | A15:LiQ | Yb | Ag | 3.78 | **9.1** |
| Comparative device 3 | F5 | F6:LiQ | LiQ | Mg:Ag | 3.34 | 6.8 |

### Technical Effect of the invention

As it may be taken from the Table 2, tested compounds of formula (I) implemented in a state-of-art semiconducting material doped with LiQ showed better results (highlighted in boldface letters) in terms of improved current efficiency than the state-of-art matrix compounds F4, F5 and F6 used as reference.

While this invention has been described in connection with what is presently considered to be practical exemplary embodiments, it is to be understood that the invention is not limited to the disclosed embodiments.

## Claims

1. An organic semiconducting material comprising at least one electron transport matrix and at least one electrical n-dopant, **characterized in that** the electron transport matrix comprises at least one first matrix compound according to Chemical Formula I: 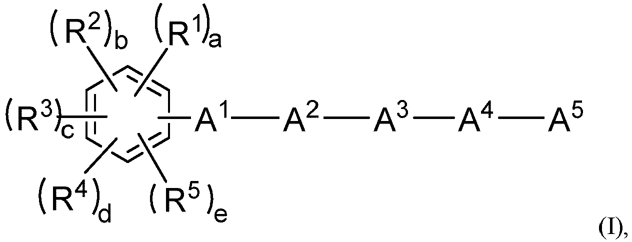 wherein
A¹, A², A³ and A⁴ is independently selected from single bond, an unsubstituted or substituted C₆ to C₃₀ arylene and an unsubstituted or substituted C₁ to C₃₀ heteroarylene;
A⁵ is selected from an unsubstituted or substituted C₆ to C₄₀ aryl group and/or from an unsubstituted or substituted C₂ to C₄₀ heteroaryl group;
R¹ to R⁵ are independently a substituted or unsubstituted C₆ to C₃₀ aryl group, a substituted or unsubstituted C₂ to C₃₀ heteroaryl group;
a to e are independently an integer of 0 or 1 and 4 ≤ a+b+c+d+e ≤ 5;
wherein, in formula (I), in the substituted group, at least one hydrogen is replaced by
(i) deuterium,
(ii) a halogen,
(iii) a C₂ to C₆₀ tertiary amino group, wherein the nitrogen atom of the tertiary amino group is substituted with two independently selected C₁ to C₃₀ hydrocarbyl groups or_the nitrogen atom of the C₂ to C₆₀ tertiary amino group_forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(v) a C₁ to C₂₂ silyl group,
(vi) a C₁ to C₃₀ alkyl group,
(vii) a C₁ to Cio alkylsilyl group,
(viii) a C₆ to C₂₂ arylsilyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

2. The organic semiconducting material according to claim 1, wherein the electrical n-dopant is selected from elemental metals, metal salts, metal complexes and organic radicals.

3. The organic semiconducting material according to claim 1 or 2, wherein the electrical n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,
- the lithium quinolinolate complex has the formula II, III or IV: 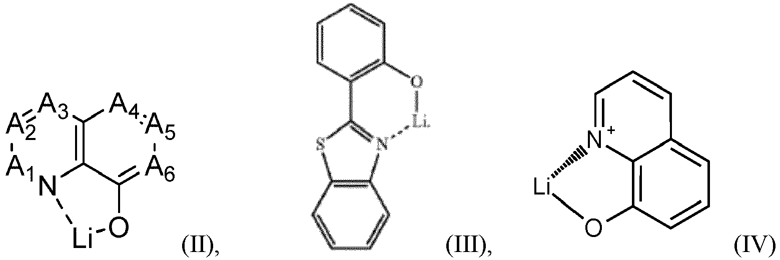 wherein
A1 to A6 are same or independently selected from CH, CR, N, O;
R is same or independently selected from hydrogen, halogen, alkyl or aryl or heteroaryl with 1 to 20 carbon atoms; and more preferred A1 to A6 are CH,
- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

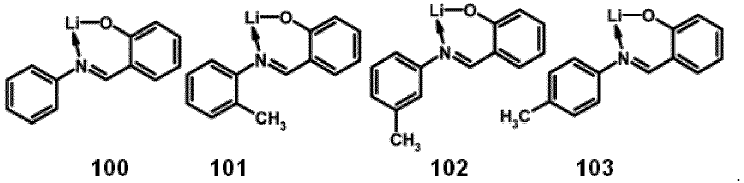

4. The organic semiconducting material according to any of the preceding claims 1 to 3, wherein the electrical n-dopant is a redox n-dopant.

5. The organic semiconducting material according to any of the preceding claims 1 to 4, wherein the redox n-dopant is selected from an elemental metal, an electrically neutral metal complex and/or an electrically neutral organic radical.

6. The organic semiconducting material according to any of the preceding claims 1 to 5, wherein the electrically neutral metal complex and/or the electrically neutral organic radical, has a redox potential which has a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple.

7. The organic semiconducting material according to any of the preceding claims 1 to 6, wherein the redox n-dopant is an electropositive elemental metal selected from alkali metals, alkaline earth metals, rare earth metals, and transition metals Ti, V, Cr and Mn; preferably from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

8. The organic semiconducting material according to any of the preceding claims 1 to 7, wherein the first matrix compound is a compound according to Chemical Formula (Ia) 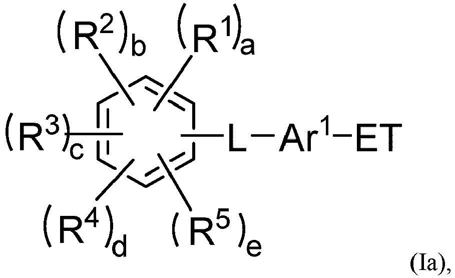 wherein, in Chemical Formula la,
Ar¹ is selected from C₆ to C₁₂ aryl and C₁ to C₁₁ heteroaryl; and
R¹ to R⁵ are independently a substituted or unsubstituted C6 to C30 aryl group, a substituted or unsubstituted C₂ to C₃₀ heteroaryl group;
a to e are independently an integer of 0 or 1 and 4 ≤ a+b+c+d+e ≤ 5;
L is a single bond, a substituted or unsubstituted C₆ to C₃₀ arylene group, or a substituted or unsubstituted C₂ to C₃₀ heteroarylene group;
ET is a unsubstituted C₆ to C₄₀ aryl or C₅ to C₄₀ heteroaryl group; or a substituted C₆ to C₄₀ aryl or C₅ to C₄₀ heteroaryl group,
wherein, in formula (Ia), in the substituted group, at least one hydrogen is replaced by
(i) deuterium,
(ii) a halogen,
(iii) a C₂ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group,
(iv) a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(v) a C₁ to C₂₂ silyl group,
(vi) a C₁ to C₃₀ alkyl group,
(vii) a C₁ to C₁₀ alkylsilyl group,
(viii) a C₆ to C₂₂ aryl silyl group,
(ix) a C₃ to C₃₀ cycloalkyl group,
(x) a C₂ to C₃₀ heterocycloalkyl group,
(xi) a C₆ to C₃₀ aryl group,
(xii) a C₂ to C₃₀ heteroaryl group,
(xiii) a C₁ to C₂₀ alkoxy group,
(xiv) a C₁ to C₃₀ perfluoro-hydrocarbyl group,
(xv) a C₁ to C₁₀ trifluoroalkyl group, or
(xvi) a cyano group.

9. The organic semiconducting material according to any of the preceding claims 1 to 8, wherein the first matrix compound is a compound according to Chemical Formula (Ib) 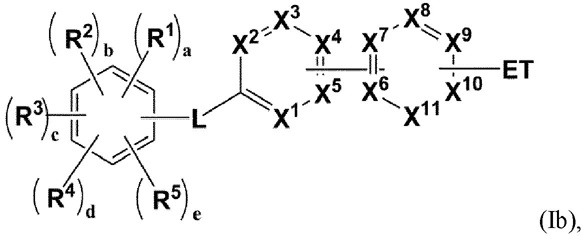 wherein in Chemical Formula Ib:
X¹ to X¹¹ are independently, N, C, or CR^{a};
R^{a} is independently, hydrogen, deuterium, a C₁ to C₃₀ alkyl group, a C₃ to C30 cycloalkyl group, a C₆ to C₃₀ aryl group, a C₆ to C₃₀ diarylamine group, a C₁ to C₃₀ alkoxy group, a C₃ to C₂₁ silyl group, a C₃ to C₂₁ silyloxy group, a C₁ to C₃₀ alkylthiol group, a C₆ to C₃₀ arylthiol group, a halogen, a C₁ to C₃₀ halogenated hydrocarbyl group, a cyano group;
R¹ to R⁵ are independently a substituted or unsubstituted C₆ to C₃₀ aryl group, a substituted or unsubstituted C₂ to C₃₀ heteroaryl group;
a to e are independently an integer of 0 or 1 and 4 ≤ a+b+c+d+e ≤ 5,
L is a single bond, a substituted or unsubstituted C₆ to C₃₀ arylene group, a substituted or unsubstituted C₂ to C₃₀ heteroarylene group, and
ET is a unsubstituted C₆ to C₄₀ aryl or C₂ to C₄₀ heteroaryl group, or a substituted C₆ to C₄₀ aryl or C₂ to C₄₀ heteroaryl group;
wherein, in formula (Ib), in the substituted group, at least one hydrogen is replaced by
(i) deuterium,
(ii) a halogen,
(iii) a C₂ to C₆₀ tertiary amino group, wherein the nitrogen atom of the C₂ to C₆₀ tertiary amino group is substituted with two independently selected C₁ to C₃₀ hydrocarbyl groups or forms a C₁ to C₃₀ heterocyclic group, a C₂ to C₆₀ phosphine oxide group, wherein the phosphorus atom of the phosphine oxide group is substituted with two C₁ to C₃₀ groups independently selected from hydrocarbyl, halogenated hydrocarbyl and hydrocarbyloxy or the phosphorus atom of the phosphine oxide group forms a C₁ to C₃₀ heterocyclic group,
(iv) a C₁ to C₂₂ silyl group,
(v) a C₁ to C₃₀ alkyl group,
(vi) a C₁ to C₁₀ alkylsilyl group,
(vii) a C₆ to C₂₂ arylsilyl group,
(viii) a C₃ to C₃₀ cycloalkyl group,
(ix) a C₂ to C₃₀ heterocycloalkyl group,
(x) a C₆ to C₃₀ aryl group,
(xi) a C₂ to C₃₀ heteroaryl group,
(xii) a C₁ to C₂₀ alkoxy group,
(xiii) a C₁ to C₃₀ perfluoro-hydrocarbyl group,
(xiv) a C₁ to C₁₀ trifluoroalkyl group, or
(xv) a cyano group.

10. The organic semiconducting material according to any of the preceding claims 1 to 9, wherein the group ET is a C₂ to C₃₀ heteroaryl group.

11. The organic semiconducting material according to any of the preceding claims 1 to 10, wherein the group ET includes at least one N, with the proviso that the group ET is not a carbazolyl group.

12. An electronic device (100) comprising a first electrode, a second electrode, and arranged between the first and second electrode, a layer of the organic semiconducting material according to any of the preceding claims 1 to 11.

13. The electronic device (100) according to claim 12, wherein the layer of the semiconducting material is a charge injection layer or a charge transport layer or a charge generating layer.

14. The electronic device (100) according to claim 12 or 13, wherein the electronic device is an electroluminescent device, preferably an organic light emitting diode.

15. A display device comprising an electronic device (100) according to any of the preceding claims 12 to 14, preferably, the display device comprises an organic light emitting diode (100) according to claim 14.

## Patentansprüche

1. Organisches halbleitendes Material, umfassend mindestens eine Elektronentransportmatrix und mindestens einen elektrischen n-Dotanden, **dadurch gekennzeichnet, dass** die Elektronentransportmatrix mindestens eine erste Matrixverbindung gemäß der Chemischen Formel I umfasst: 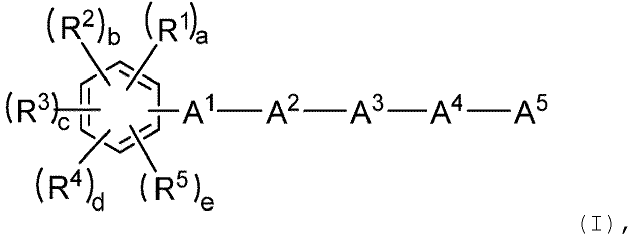 wobei
A¹, A², A³ und A⁴ unabhängig aus einer Einfachbindung, einem unsubstituierten oder substituierten C₆- bis C₃₀-Arylen und einem unsubstituierten oder substituierten C₁- bis C₃₀-Heteroarylen ausgewählt sind;
A⁵ aus einer unsubstituierten oder substituierten C₆- bis C₄₀-Arylgruppe und/oder einer unsubstituierten oder substituierten C₂-bis C₄₀-Heteroarylgruppe ausgewählt ist;
R¹ bis R⁵ unabhängig für eine substituierte oder unsubstituierte C₆- bis C₃₀-Arylgruppe oder eine substituierte oder unsubstituierte C₂- bis C₃₀-Heteroarylgruppe stehen;
a bis e unabhängig für eine ganze Zahl mit einem Wert von 0 oder 1 stehen und 4 ≤ a + b + c + d + e ≤ 5;
wobei in Formel (I) in der substituierten Gruppe mindestens ein Wasserstoff durch
(i) Deuterium,
(ii) ein Halogen,
(iii) eine tertiäre C₂- bis C₆₀-Aminogruppe, wobei das Stickstoffatom der tertiären Aminogruppe durch zwei unabhängig ausgewählte C₁- bis C₃₀-Hydrocarbylgruppen substituiert ist oder das Stickstoffatom der tertiären C₂- bis C₆₀-Aminogruppe eine C₁- bis C₃₀-Heterocyclylgruppe bildet,
(iv) eine C₂- bis C₆₀-Phosphinoxidgruppe, wobei das Phosphoratom der Phosphinoxidgruppe durch zwei unabhängig ausgewählte C₁- bis C₃₀-Gruppen, die unabhängig aus Hydrocarbyl, halogeniertem Hydrocarbyl und Hydrocarbyloxy ausgewählt sind, substituiert ist oder das Phosphoratom der Phosphinoxidgruppe eine C₁- bis C₃₀-Heterocyclylgruppe bildet,
(v) eine C₁- bis C₂₂-Silylgruppe,
(vi) eine C₁- bis C₃₀-Alkylgruppe,
(vii) eine C₁- bis C₁₀-Alkylsilylgruppe,
(viii) eine C₆- bis C₂₂-Arylsilylgruppe,
(ix) eine C₃- bis C₃₀-Cycloalkylgruppe,
(x) eine C₂- bis C₃₀-Heterocycloalkylgruppe,
(xi) eine C₆- bis C₃₀-Arylgruppe,
(xii) eine C₂- bis C₃₀-Heteroarylgruppe,
(xiii) eine C₁- bis C₂₀-Alkoxygruppe,
(xiv) eine C₁- bis C₃₀-Perfluorhydrocarbylgruppe,
(xv) eine C₁- bis C₁₀-Trifluoralkylgruppe oder
(xvi) eine Cyanogruppe
ersetzt ist.

2. Organisches halbleitendes Material nach Anspruch 1, wobei der elektrische n-Dotand aus elementaren Metallen, Metallsalzen, Metallkomplexen und organischen Radikalen ausgewählt ist.

3. Organisches halbleitendes Material nach Anspruch 1 oder 2, wobei der elektrische n-Dotand aus Alkalimetallsalzen und Alkalimetallkomplexen, vorzugsweise aus Lithiumsalzen und organischen Lithiumkomplexen, weiter bevorzugt aus Lithiumhalogeniden und organischen Lithiumchelaten, noch weiter bevorzugt aus Lithiumfluorid, einem Lithiumchinolinolat, Lithiumborat, Lithiumphenolat, Lithiumpyridinolat oder einem Lithiumkomplex mit einem Schiff-Base-Liganden, ausgewählt ist; ganz besonders bevorzugt
der Lithiumchinolinolatkomplex die Formel II, III oder IV aufweist: 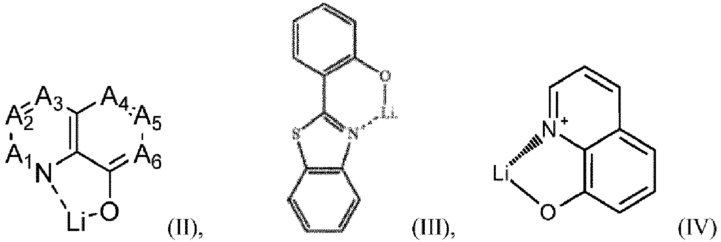 wobei
A₁ bis A₆ gleich oder unabhängig aus CH, CR, N und O ausgewählt sind;
R gleich oder unabhängig aus Wasserstoff, Halogen, Alkyl oder Aryl oder Heteroaryl mit 1 bis 20 Kohlenstoffatomen ausgewählt ist; und weiter bevorzugt A₁ bis A₆ für CH stehen,
- es sich bei dem organischen Liganden auf Borat-Basis um ein Tetra(1H-pyrazol-1-yl)borat handelt,
- es sich bei dem Phenolat um ein 2- (Pyridin-2-yl)phenolat, ein 2-(Diphenylphosphoryl)phenolat, ein Imidazolphenolat, 2-(Pyridin-2-yl)phenolat oder 2-(1-Phenyl-1H-benzo[d]imidazol-2-yl)phenolat handelt,
- es sich bei dem Pyridinolat um ein 2-(Diphenylphosphoryl)pyridin-3-olat handelt,
- die Lithium-Schiff-Base die Struktur 100, 101, 102 oder 103 aufweist:

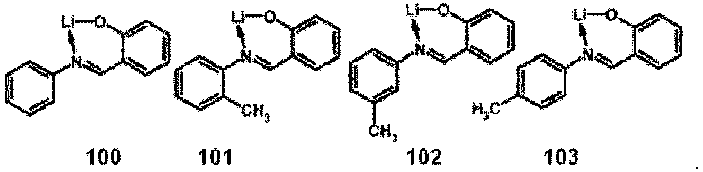

4. Organisches halbleitendes Material nach einem der vorhergehenden Ansprüche 1 bis 3, wobei es sich bei dem elektrischen n-Dotanden um einen Redox-n-Dotanden handelt.

5. Organisches halbleitendes Material nach einem der vorhergehenden Ansprüche 1 bis 4, wobei der Redox-n-Dotand aus einem elementaren Metall, einem elektrisch neutralen Metallkomplex und/oder einem elektrisch neutralen organischen Radikal ausgewählt ist.

6. Organisches halbleitendes Material nach einem der vorhergehenden Ansprüche 1 bis 5, wobei der elektrisch neutrale Metallkomplex und/oder das elektrisch neutrale organische Radikal ein Redoxpotential aufweist, das bei Messung durch Cyclovoltammetrie gegen ein Ferrocen/Ferrocenium-Referenzredoxpaar einen Wert aufweist, der negativer als -0,5 V, vorzugsweise negativer als -1,2 V, weiter bevorzugt negativer als -1,7 V, noch weiter bevorzugt negativer als -2,1 V, ganz besonders bevorzugt negativer als -2,5 V ist.

7. Organisches halbleitendes Material nach einem der vorhergehenden Ansprüche 1 bis 6, wobei es sich bei dem Redox-n-Dotanden um ein elektropositives elementares Metall handelt, das aus Alkalimetallen, Erdalkalimetallen, Seltenerdmetallen und Übergangsmetallen Ti, V, Cr und Mn, vorzugsweise aus Li, Na, K, Rb, Cs, Mg, Ca, Sr, Ba, Sm, Eu, Tm und Yb; weiter bevorzugt aus Li, Na, K, Rb, Cs, Mg und Yb, noch weiter bevorzugt aus Li, Na, Cs und Yb und ganz besonders bevorzugt aus Li, Na und Yb ausgewählt ist.

8. Organisches halbleitendes Material nach einem der vorhergehenden Ansprüche 1 bis 7, wobei sich bei der ersten Matrixverbindung eine Verbindung gemäß der Chemischen Formel (Ia) handelt: 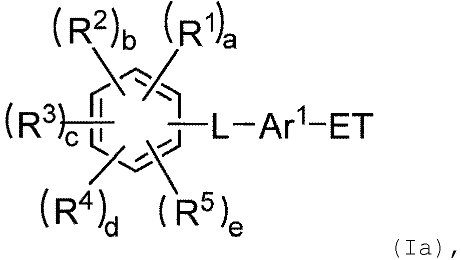 wobei in der Chemischen Formel Ia
Ar¹ aus C6- bis C₁₂-Aryl und C₁- bis C₁₁-Heteroaryl ausgewählt ist und
R¹ bis R⁵ unabhängig für eine substituierte oder unsubstituierte C₆- bis C₃₀-Arylgruppe oder eine substituierte oder unsubstituierte C₂- bis C₃₀-Heteroarylgruppe stehen;
a bis e unabhängig für eine ganze Zahl mit einem Wert von 0 oder 1 stehen und 4 ≤ a + b + c + d + e ≤ 5;
L für eine Einfachbindung, eine substituierte oder unsubstituierte C₆- bis C₃₀-Arylengruppe oder eine substituierte oder unsubstituierte C₂- bis C₃₀-Heteroarylengruppe steht;
ET für eine unsubstituierte C₆- bis C₄₀-Arylgruppe oder C₅- bis C₄₀-Heteroarylgruppe oder eine substituierte C₆- bis C₄₀-Arylgruppe oder C₅- bis C₄₀-Heteroarylgruppe steht,
wobei in Formel (Ia) in der substituierten Gruppe mindestens ein Wasserstoff durch
(i) Deuterium,
(ii) ein Halogen,
(iii) eine tertiäre C₂- bis C₆₀-Aminogruppe, wobei das Stickstoffatom der tertiären Aminogruppe durch zwei unabhängig ausgewählte C₁- bis C₃₀-Hydrocarbylgruppen substituiert ist oder das Stickstoffatom der tertiären C₂- bis C₆₀-Aminogruppe eine C₁- bis C₃₀-Heterocyclylgruppe bildet,
(iv) eine C₂- bis C₆₀-Phosphinoxidgruppe, wobei das Phosphoratom der Phosphinoxidgruppe durch zwei unabhängig ausgewählte C₁- bis C₃₀-Gruppen, die unabhängig aus Hydrocarbyl, halogeniertem Hydrocarbyl und Hydrocarbyloxy ausgewählt sind, substituiert ist oder das Phosphoratom der Phosphinoxidgruppe eine C₁- bis C₃₀-Heterocyclylgruppe bildet,
(v) eine C₁- bis C₂₂-Silylgruppe,
(vi) eine C₁- bis C₃₀-Alkylgruppe,
(vii) eine C₁- bis C₁₀-Alkylsilylgruppe,
(viii) eine C₆- bis C₂₂-Arylsilylgruppe,
(ix) eine C₃- bis C₃₀-Cycloalkylgruppe,
(x) eine C₂- bis C₃₀-Heterocycloalkylgruppe,
(xi) eine C₆- bis C₃₀-Arylgruppe,
(xii) eine C₂- bis C₃₀-Heteroarylgruppe,
(xiii) eine C₁- bis C₂₀-Alkoxygruppe,
(xiv) eine C₁- bis C₃₀-Perfluorhydrocarbylgruppe,
(xv) eine C₁- bis C₁₀-Trifluoralkylgruppe oder
(xvi) eine Cyanogruppe
ersetzt ist.

9. Organisches halbleitendes Material nach einem der vorhergehenden Ansprüche 1 bis 8, wobei sich bei der ersten Matrixverbindung eine Verbindung gemäß der Chemischen Formel (Ib) handelt: 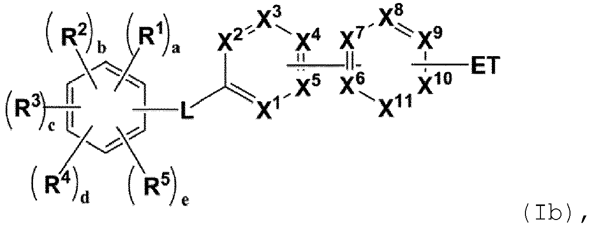 wobei in der Chemischen Formel Ib
X¹ bis X¹¹ unabhängig für N, C oder CR^{a} stehen;
R^{a} unabhängig für Wasserstoff, Deuterium, eine C₁- bis C₃₀-Alkylgruppe,eine C₃- bis C₃₀-Cycloalkylgruppe, eine C₆- bis C₃₀-Arylgruppe, eine C₆- bis C₃₀-Diarylamingruppe, eine C₁- bis C₃₀-Alkoxygruppe, eine C₃- bis C₂₁-Silylgruppe, eine C₃- bis C₂₁-Silyloxygruppe, eine C₁- bis C₃₀-Alkylthiolgruppe, eine C₆- bis C₃₀-Arylt-hiolgruppe, ein Halogen, eine halogenierte C₁-bis C₃₀-Hydrocarbylgruppe oder eine Cyanogruppe steht;
R¹ bis R⁵ unabhängig für eine substituierte oder unsubstituierte C₆- bis C₃₀-Arylgruppe oder eine substituierte oder unsubstituierte C₂- bis C₃₀-Heteroarylgruppe stehen;
a bis e unabhängig für eine ganze Zahl mit einem Wert von 0 oder 1 stehen und 4 ≤ a + b + c + d + e ≤ 5;
L für eine Einfachbindung, eine substituierte oder unsubstituierte C₆- bis C₃₀-Arylengruppe oder eine substituierte oder unsubstituierte C₂- bis C₃₀-Heteroarylengruppe steht;
ET für eine unsubstituierte C₆- bis C₄₀-Arylgruppe oder C₂- bis C₄₀-Heteroarylgruppe oder eine substituierte C₆- bis C₄₀-Arylgruppe oder C₂- bis C₄₀-Heteroarylgruppe steht,
wobei in Formel (Ib) in der substituierten Gruppe mindestens ein Wasserstoff durch
(i) Deuterium,
(ii) ein Halogen,
(iii) eine tertiäre C₂- bis C₆₀-Aminogruppe, wobei das Stickstoffatom der tertiären C₂-bis C₆₀-Aminogruppe durch zwei unabhängig ausgewählte C₁- bis C₃₀-Hydrocarbylgruppen substituiert ist oder eine C₁- bis C₃₀-Heterocyclylgruppe bildet, eine C₂- bis C₆₀-Phosphinoxidgruppe, wobei das Phosphoratom der Phosphinoxidgruppe durch zwei C₁- bis C₃₀-Gruppen, die unabhängig aus Hydrocarbyl, halogeniertem Hydrocarbyl und Hydrocarbyloxy ausgewählt sind, substituiert ist oder das Phosphoratom der Phosphinoxidgruppe eine C₁- bis C₃₀-Heterocyclylgruppe bildet,
(iv) eine C₁- bis C₂₂-Silylgruppe,
(v) eine C₁- bis C₃₀-Alkylgruppe,
(vi) eine C₁- bis C₁₀-Alkylsilylgruppe,
(vii) eine C₆- bis C₂₂-Arylsilylgruppe,
(viii) eine C₃- bis C₃₀-Cycloalkylgruppe,
(ix) eine C₂- bis C₃₀-Heterocycloalkylgruppe,
(x) eine C₆- bis C₃₀-Arylgruppe,
(xi) eine C₂- bis C₃₀-Heteroarylgruppe,
(xii) eine C₁- bis C₂₀-Alkoxygruppe,
(xiii) eine C₁- bis C₃₀-Perfluorhydrocarbylgruppe,
(xiv) eine C₁- bis C₁₀-Trifluoralkylgruppe oder
(xv) eine Cyanogruppe
ersetzt ist.

10. Organisches halbleitendes Material nach einem der vorhergehenden Ansprüche 1 bis 9, wobei die Gruppe ET für eine C₂- bis C₃₀-Heteroarylgruppe steht.

11. Organisches halbleitendes Material nach einem der vorhergehenden Ansprüche 1 bis 10, wobei die Gruppe ET mindestens ein N enthält, mit der Maßgabe, dass es sich bei der Gruppe ET nicht um eine Carbazolylgruppe handelt.

12. Elektronische Vorrichtung (100) mit einer ersten Elektrode, einer zweiten Elektrode und einer zwischen der ersten und zweiten Elektrode angeordneten Schicht des organischen halbleitenden Materials nach einem der vorhergehenden Ansprüche 1 bis 11.

13. Elektronische Vorrichtung (100) nach Anspruch 12, wobei es sich bei der Schicht aus dem halbleitenden Material um eine Ladungsinjektionsschicht oder eine Ladungstransportschicht oder eine Ladungserzeugungsschicht handelt.

14. Elektronische Vorrichtung (100) nach Anspruch 12 oder 13, wobei es sich bei der elektronischen Vorrichtung um eine Elektrolumineszenzvorrichtung, vorzugsweise eine organische Leuchtdiode, handelt.

15. Anzeigevorrichtung, umfassend eine elektronische Vorrichtung (100) nach einem der vorhergehenden Ansprüche 12 bis 14, wobei die Anzeigevorrichtung vorzugsweise eine organische Leuchtdiode (100) nach Anspruch 14 umfasst.

## Revendications

1. Matériau semiconducteur organique comprenant au moins une matrice de transport d'électrons et au moins un dopant de type n électrique, **caractérisé en ce que** la matrice de transport d'électrons comprend au moins un premier composé matriciel répondant à la formule chimique I : 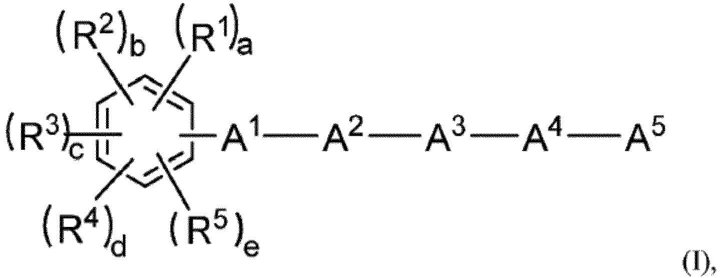 dans lequel
A¹, A², A³ et A⁴ sont indépendamment choisis parmi une liaison simple, un groupe arylène en C₆ à C₃₀ non substitué ou substitué et un groupe hétéroarylène en C₁ à C₃₀ non substitué ou substitué ;
A⁵ est choisi parmi un groupe aryle en C₆ à C₄₀ non substitué ou substitué et/ou parmi un groupe hétéroaryle en C₂ à C₄₀ non substitué ou substitué ;
R¹ à R⁵ représentent indépendamment un groupe aryle en C₆ à C₃₀ substitué ou non substitué, un groupe hétéroaryle en C₂ à C₃₀ substitué ou non substitué ;
a à e représentent indépendamment un nombre entier valant 0 ou 1 et 4 ≤ a+b+c+d+e ≤ 5 ;
dans lequel, dans la formule (I), dans le groupe substitué, au moins un atome d'hydrogène est remplacé par
(i) un atome de deutérium,
(ii) un atome d'halogène,
(iii) un groupe amino tertiaire en C₂ à C₆₀, l'atome d'azote du groupe amino tertiaire étant substitué par deux groupes hydrocarbyle en C₁ à C₃₀ indépendamment choisis ou l'atome d'azote du groupe amino tertiaire en C₂ à C₆₀ formant un groupe hétérocyclique en C₁ à C₃₀,
(iv) un groupe oxyde de phosphine en C₂ à C₆₀, l'atome de phosphore du groupe oxyde de phosphine étant substitué par deux groupes en C₁ à C₃₀ indépendamment choisis parmi un hydrocarbyle, un hydrocarbyle halogéné et un hydroxycarbyloxy ou l'atome de phosphore du groupe oxyde de phosphine formant un groupe hétérocyclique en C₁ à C₃₀,
(v) un groupe silyle en C₁ à C₂₂,
(vi) un groupe alkyle en C₁ à C₃₀,
(vii) un groupe alkylsilyle en C₁ à C₁₀,
(viii) un groupe arylsilyle en C₆ à C₂₂,
(ix) un groupe cycloalkyle en C₃ à C₃₀,
(x) un groupe hétérocycloalkyle en C₂ à C₃₀,
(xi) un groupe aryle en C₆ à C₃₀,
(xii) un groupe hétéroaryle en C₂ à C₃₀,
(xiii) un groupe alcoxy en C₁ à C₂₀,
(xiv) un groupe perfluorohydrocarbyle en C₁ à C₃₀,
(xv) un groupe trifluoroalkyle en C₁ à C₁₀, ou
(xvi) un groupe cyano.

2. Matériau semiconducteur organique selon la revendication 1, dans lequel le dopant de type n électrique est choisi parmi les métaux élémentaires, les sels métalliques, les complexes métalliques et les radicaux organiques.

3. Matériau semiconducteur organique selon la revendication 1 ou 2, dans lequel le dopant de type n électrique est choisi parmi les sels de métal alcalin et les complexes de métal alcalin ; de préférence parmi les sels de lithium et les complexes organiques de lithium ; plus préférablement parmi les halogénures de lithium et les chélates organiques de lithium ; encore plus préférablement parmi le fluorure de lithium, un quinolinoate de lithium, un borate de lithium, un phénolate de lithium, un pyridinolate de lithium ou parmi un complexe à base de lithium avec un ligand de type base de Schiff ; de manière préférée entre toutes,
- le complexe de quinolinolate de lithium a la formule II, III ou IV : 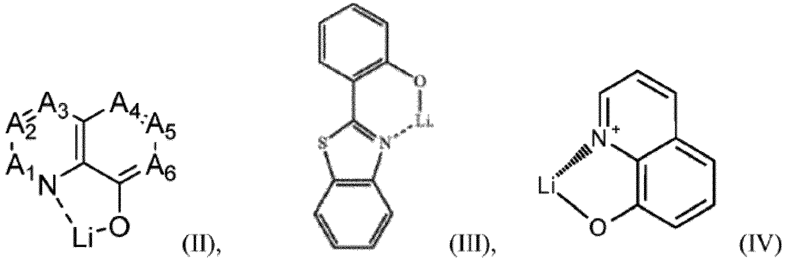 dans lequel
A₁ à A6 sont identiques ou choisis de manière indépendante parmi CH, CR, N, O ;
R est identique ou choisi de manière indépendante parmi un atome d'hydrogène, un atome d'halogène, un groupe alkyle ou aryle ou hétéroaryle comprenant 1 à 20 atomes de carbone ; et plus préférablement A₁ à A6 représentent CH,
- le ligand organique à base de borate est un tétra(1H-pyrazol-1-yl)borate,
- le phénolate est un 2-(pyridin-2-yl)phénolate, un 2-(diphénylphosphoryl)phénolate, un imidazolphénolate, un 2-(pyridin-2-yl)phénolate ou un 2-(1-phényl-1H-benzo[d]imidazol-2-yl)phénolate,
- le pyridinolate est un 2-(diphénylphosphoryl)pyridin-3-olate,
- la base de Schiff à base de lithium a la structure 100, 101, 102 ou 103 :

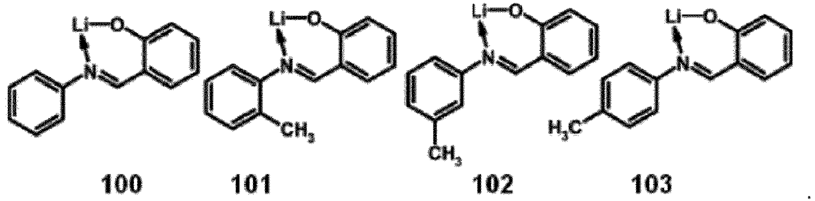

4. Matériau semiconducteur organique selon l'une quelconque des revendications 1 à 3, dans lequel le dopant de type n électrique est un dopant de type n redox.

5. Matériau semiconducteur organique selon l'une quelconque des revendications 1 à 4, dans lequel le dopant de type n redox est choisi parmi un métal élémentaire, un complexe métallique électriquement neutre et/ou un radical organique électriquement neutre.

6. Matériau semiconducteur organique selon l'une quelconque des revendications 1 à 5, dans lequel le complexe métallique électriquement neutre et/ou le radical organique électriquement neutre a un potentiel redox qui a une valeur qui est plus négative que -0,5 V, de préférence plus négative que -1,2 V, plus préférablement plus négative que -1,7 V, encore plus préférablement plus négative que -2,1 V, de manière préférée entre toutes plus négative que -2,5 V, si elle est mesurée par voltampérométrie cyclique par rapport à un couple redox de référence ferrocène/ferrocénium.

7. Matériau semiconducteur organique selon l'une quelconque des revendications 1 à 6, dans lequel le dopant de type n redox est un métal élémentaire électropositif choisi parmi les métaux alcalins, les métaux alcalino-terreux, les métaux de terre rare et les métaux de transition Ti, V, Cr et Mn ; de préférence parmi Li, Na, K, Rb, Cs, Mg, Ca, Sr, Ba, Sm, Eu, Tm, Yb ; plus préférablement parmi Li, Na, K, Rb, Cs, Mg et Yb, encore plus préférablement parmi Li, Na, Cs et Yb, de manière préférée entre toute parmi Li, Na et Yb.

8. Matériau semiconducteur organique selon l'une quelconque des revendications 1 à 7, dans lequel le premier composé matriciel est un composé conformément à la formule chimique (Ia) 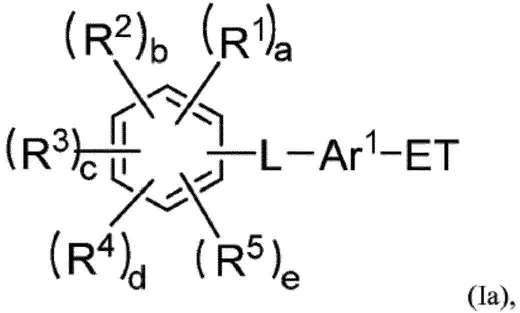 dans lequel, dans la formule chimique la,
Ar¹ est choisi parmi un groupe aryle en C₆ à C₁₂ et un groupe hétéroaryle en C₁ à C₁₁ ; et
R¹ à R⁵ représentent indépendamment un groupe aryle en C₆ à C₃₀ substitué ou non substitué, un groupe hétéroaryle en C₂ à C₃₀ substitué ou non substitué ;
a à e représentent indépendamment un nombre entier valant 0 ou 1 et 4 ≤ a+b+c+d+e ≤ 5 ;
L représente une liaison simple, un groupe arylène en C₆ à C₃₀ substitué ou non substitué ou un groupe hétéroarylène en C₂ à C₃₀ substitué ou non substitué ;
ET représente un groupe aryle en C₆ à C₄₀ ou hétéroaryle en C₅ à C₄₀ non substitué ; ou un groupe aryle en C₆ à C₄₀ ou hétéroaryle en C₅ à C₄₀ substitué,
dans lequel, dans la formule (Ia), dans le groupe substitué, au moins un atome d'hydrogène est remplacé par
(i) un atome de deutérium,
(ii) un atome d'halogène,
(iii) un groupe amino tertiaire en C₂ à C₆₀, l'atome d'azote du groupe amino tertiaire en C₂ à C₆₀ étant substitué par deux groupes hydrocarbyle en C₁ à C₃₀ indépendamment choisis ou formant un groupe hétérocyclique en C₁ à C₃₀,
(iv) un groupe oxyde de phosphine en C₂ à C₆₀, l'atome de phosphore du groupe oxyde de phosphine étant substitué par deux groupes en C₁ à C₃₀ indépendamment choisis parmi un hydrocarbyle, un hydrocarbyle halogéné et un hydroxycarbyloxy ou l'atome de phosphore du groupe oxyde de phosphine formant un groupe hétérocyclique en C₁ à C₃₀,
(v) un groupe silyle en C₁ à C₂₂,
(vi) un groupe alkyle en C₁ à C₃₀,
(vii) un groupe alkylsilyle en C₁ à C₁₀,
(viii) un groupe arylsilyle en C₆ à C₂₂,
(ix) un groupe cycloalkyle en C₃ à C₃₀,
(x) un groupe hétérocycloalkyle en C₂ à C₃₀,
(xi) un groupe aryle en C₆ à C₃₀,
(xii) un groupe hétéroaryle en C₂ à C₃₀,
(xiii) un groupe alcoxy en C₁ à C₂₀,
(xiv) un groupe perfluorohydrocarbyle en C₁ à C₃₀,
(xv) un groupe trifluoroalkyle en C₁ à C₁₀, ou
(xvi) un groupe cyano.

9. Matériau semiconducteur organique selon l'une quelconque des revendications 1 à 8, dans lequel le premier composé matriciel est un composé conformément à la formule chimique (Ib) 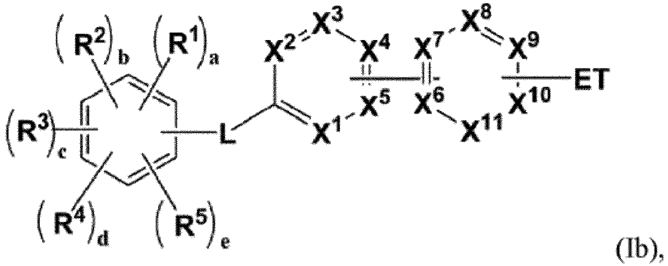 dans lequel, dans la formule chimique Ib :
X¹ à X¹¹ représentent indépendamment N, C ou CR^{a} ;
R^{a} représente indépendamment un atome d'hydrogène, un atome de deutérium, un groupe alkyle en C₁ à C₃₀, un groupe cycloalkyle en C₃ à C₃₀, un groupe aryle en C₆ à C₃₀, un groupe diarylamine en C₆ à C₃₀, un groupe alcoxy en C₁ à C₃₀, un groupe silyle en C₃ à C₂₁, un groupe silyloxy en C₃ à C₂₁, un groupe alkylthiol en C₁ à C₃₀, un groupe arylthiol en C₆ à C₃₀, un atome d'halogène, un groupe hydrocarbyle halogéné en C₁ à C₃₀, un groupe cyano ;
R¹ à R⁵ représentent indépendamment un groupe aryle en C₆ à C₃₀ substitué ou non substitué, un groupe hétéroaryle en C₂ à C₃₀ substitué ou non substitué ;
a à e représentent indépendamment un nombre entier valant 0 ou 1 et 4 ≤ a+b+c+d+e ≤ 5 ;
L représente une liaison simple, un groupe arylène en C₆ à C₃₀ substitué ou non substitué, un groupe hétéroarylène en C₂ à C₃₀ substitué ou non substitué, et
ET représente un groupe aryle en C₆ à C₄₀ ou hétéroaryle en C₂ à C₄₀ non substitué ou un groupe aryle en C₆ à C₄₀ ou hétéroaryle en C₂ à C₄₀ substitué ;
dans lequel dans la formule (Ib), dans le groupe substitué, au moins un atome d'hydrogène est remplacé par
(i) un atome de deutérium,
(ii) un atome d'halogène,
(iii) un groupe amino tertiaire en C₂ à C₆₀, l'atome d'azote du groupe amino tertiaire en C₂ à C₆₀ étant substitué par deux groupes hydrocarbyle en C₁ à C₃₀ indépendamment choisis ou formant un groupe hétérocyclique en C₁ à C₃₀, un groupe oxyde de phosphine en C₂ à C₆₀, l'atome de phosphore du groupe oxyde de phosphine étant substitué par deux groupes en C₁ à C₃₀ indépendamment choisis parmi un hydrocarbyle, un hydrocarbyle halogéné et un hydroxycarbyloxy ou l'atome de phosphore du groupe oxyde de phosphine formant un groupe hétérocyclique en C₁ à C₃₀,
(iv) un groupe silyle en C₁ à C₂₂,
(v) un groupe alkyle en C₁ à C₃₀,
(vi) un groupe alkylsilyle en C₁ à C₁₀,
(vii) un groupe arylsilyle en C₆ à C₂₂,
(viii) un groupe cycloalkyle en C₃ à C₃₀,
(ix) un groupe hétérocycloalkyle en C₂ à C₃₀,
(x) un groupe aryle en C₆ à C₃₀,
(xi) un groupe hétéroaryle en C₂ à C₃₀,
(xii) un groupe alcoxy en C₁ à C₂₀,
(xiii) un groupe perfluorohydrocarbyle en C₁ à C₃₀,
(xiv) un groupe trifluoroalkyle en C₁ à C₁₀, ou
(xv) un groupe cyano.

10. Matériau semiconducteur organique selon l'une quelconque des revendications 1 à 9, dans lequel le groupe ET représente un groupe hétéroaryle en C₂ à C₃₀.

11. Matériau semiconducteur organique selon l'une quelconque des revendications 1 à 10, dans lequel le groupe ET comprend au moins un N, à condition que le groupe ET ne soit pas un groupe carbazoyle.

12. Dispositif électronique (100) comprenant une première électrode, une seconde électrode et agencée entre la première et la seconde électrode, une couche du matériau semiconducteur organique selon l'une quelconque des revendications 1 à 11.

13. Dispositif électronique (100) selon la revendication 12, dans lequel la couche du matériau semiconducteur est une couche d'injection de charge ou une couche de transport de charge ou une couche de génération de charge.

14. Dispositif électronique (100) selon la revendication 12 ou 13, dans lequel le dispositif électronique est un dispositif électroluminescent, de préférence une diode électroluminescente organique.

15. Dispositif d'affichage comprenant un dispositif électronique (100) selon l'une quelconque des revendications 12 à 14, de préférence, le dispositif d'affichage comprend une diode électroluminescente organique (100) selon la revendication 14.
